# EUROPEAN PATENT SPECIFICATION

(11) **EP 3 962 389 B1**
(45) Date of publication and mention of the grant of the patent: **07.06.2023**
(21) Application number: 20705680.5
(22) Date of filing: 14.02.2020
(51) Int. Cl.: A61B 18/18

(54) **ELECTROSURGICAL SYSTEM**
ELEKTROCHIRURGISCHES SYSTEM
SYSTÈME ÉLECTROCHIRURGICAL

(30) Priority: 29.04.2019 GB 201905926
(43) Date of publication of application: 09.03.2022
(73) Proprietor: Creo Medical Limited, Chepstow, Wales NP16 5UH (GB)
(72) Inventor: HANCOCK, Christopher Paul, Chepstow, Wales NP16 5UH (GB)
(74) Representative: Mewburn Ellis LLP
(86) International application number: PCT/EP2020/053916
(87) International publication number: WO 2020/221485

(56) References cited:
- US-A1- 2012 191 072
- US-A1- 2015 112 190
- US-A1- 2018 047 555

## Description

### FIELD OF THE INVENTION

The invention relates to an electrosurgical system for supplying microwave energy to biological tissue and for performing electroporation on biological tissue. In particular, the electrosurgical system includes an electrosurgical instrument having a conduit for extracting biological tissue from a treatment region, and a cytometer or cell sorter for identifying the presence of a particular cell type in the extracted biological tissue. The electrosurgical system may be arranged to ablate tissue, such as a tumour.

### BACKGROUND TO THE INVENTION

Electromagnetic (EM) energy, and in particular microwave and radiofrequency (RF) energy, has been found to be useful in electrosurgical operations, for its ability to cut, coagulate, and ablate body tissue. Typically, systems for delivering EM energy to body tissue include a generator comprising a source of EM energy, and an electrosurgical instrument connected to the generator, for delivering the energy to tissue. In this context, reference is made to document US 2012/191072 A1. Conventional electrosurgical instruments are often designed to be inserted percutaneously into the patient's body. However, it can be difficult to locate the instrument percutaneously in the body, for example if the target site is in a moving lung or a thin walled section of the gastrointestinal (GI) tract. Other electrosurgical instruments can be delivered to a target site by a surgical scoping device (e.g. an endoscope) which can be run through channels in the body such as airways or the lumen of the oesophagus or colon. This allows for minimally invasive treatments, which can reduce the mortality rate of patients and reduce intraoperative and postoperative complication rates.

Tissue ablation using microwave EM energy is based on the fact that biological tissue is largely composed of water. Human soft organ tissue is typically between 70% and 80% water content. Water molecules have a permanent electric dipole moment, meaning that a charge imbalance exists across the molecule. This charge imbalance causes the molecules to move in response to the forces generated by application of a time varying electric field as the molecules rotate to align their electric dipole moment with the polarity of the applied field. At microwave frequencies, rapid molecular oscillations result in frictional heating and consequential dissipation of the field energy in the form of heat. This is known as dielectric heating.

This principle is harnessed in microwave ablation therapies, where water molecules in target tissue are rapidly heated by application of a localised electromagnetic field at microwave frequencies, resulting in tissue coagulation and cell death. It is known to use microwave emitting electrosurgical instruments to treat various conditions in the lungs and other organs. For example, in the lungs, microwave radiation can be used to treat asthma and ablate tumours or lesions.

Another type of tumour treatment makes use of an effect known as electroporation (or electropermeabilization). In this technique, electrical pulses are applied to biological tissue to cause nanoscale pores to open in cell membranes at a target site. The pores permit anticancer drugs or other material that cannot normally permeate through the cell membrane to enter the cells. The pores may then reseal to trap the material within the cell, where it may cause a therapeutic effect (e.g. to kill the cell). It is also known to use electroporation to create permanent nanoscale pores in the cell membrane. These pores do not reseal, and thus disrupt cell homeostasis, eventually leading to cell death. This technique is known as irreversible electroporation or non-thermal irreversible electroporation. Unlike thermal ablation, e.g. using microwave energy, irreversible electroporation preserves the extracellular matrix.

Dielectrophoresis (DEP) is a phenomenon in which a force is exerted on a dielectric particle when it is subjected to a non-uniform electric field. This force does not require the particle to be charged. All particles exhibit dielectrophoretic activity in the presence of electric fields. However, the strength of the force depends strongly on the medium and particles electrical properties, on the particles shape and size, as well as on the frequency of the electric field. Consequently, fields of a particular frequency can manipulate particles with great selectivity. Dielectrophoresis can be used to manipulate, transport, separate and sort different types of particles. Since biological cells have dielectric properties, dielectrophoresis has medical applications. For example, instruments that separate cancer cells from healthy cells have been made.

The present invention has been devised in light of the above considerations.

### SUMMARY OF THE INVENTION

The invention is defined in the appended set of claims. At its most general, the invention provides an electrosurgical system for supplying microwave energy to biological tissue and for performing electroporation on biological tissue. The system includes an electrosurgical instrument for extracting biological tissue from a treatment region at a distal end of the instrument. The system includes a cytometer or cell sorter for identifying the presence of a particular cell type or category (e.g. cancerous, healthy, cancer stem cell) in the extracted biological tissue. The system is further configured to radiate microwave energy from the instrument into the treatment region, and to establish an electric field at the instrument for electroporation of biological tissue in the treatment region. The microwave energy may be used to perform tissue measurements, tissue ablation or activation of drugs (e.g. tissue heating without ablating). The electroporation performed may be reversible (aka temporary) electroporation or non-thermal irreversible (aka permanent) electroporation.

The electrosurgical instrument may have a radiating tip portion capable performing tissue ablation using microwave energy and electroporation in a minimally invasive manner. The electrosurgical instrument may be used to perform microwave ablation and electroporation separately (e.g. sequentially) or simultaneously. The radiating tip portion may be dimensioned to be suitable for insertion into a pancreas via a surgical scoping device, to provide a rapid and accurate alternative to known RF ablation techniques. By enabling tumours within the pancreas to be treated using a minimally invasive procedure, it may be a viable option to use ablation and/or electroporation treatment for both curative as well as palliative reasons.

Although the invention may find particular use in the pancreas, it may also be suitable for use in other awkward treatment sites, such as the lungs, brain, etc. The instrument structure disclosed herein enables the radiating tip portion to be provided with appropriate length and rigidity for use in a variety of settings.

By combining the ability to perform microwave ablation and electroporation with the same instrument, it is possible to rapidly change between treatment modalities during an electrosurgical procedure without having to change instruments. Microwave ablation and electroporation may be used in a complimentary manner, in order to treat target tissue more effectively and/or minimise treatment time. Due to the small diameter of the radiating tip portion, the radiating tip portion may heat up when it is used to deliver microwave energy into tissue. Excessive heating may cause damage to healthy surrounding tissue, so it is often necessary to wait after application of microwave energy for the radiating tip portion to cool back down. With the instrument of the invention, it is possible to alternate between treatment with microwave energy and electroporation, in order to avoid excessive heating of the radiating tip portion. This may enable the overall treatment time to be minimised.

The electrosurgical instrument also has the ability to perform tissue extraction or tissue biopsy. In this way, biological tissue can be obtained from a treatment region for examination by a cytometer or cell sorter. For example, the examination may involve determining whether or not the biological tissue contains one or more predetermined cell types, such as, for example: healthy cells, cancerous cells, cancer stem cells. In an embodiment, the cytometer may be able to distinguish between two or more different cell types or categories, for example, between healthy cells and cancer cells, or between cancer stem cells and cancer cells. Accordingly, the cytometer can be used to identify the presence of a tumour. Also, on detecting cancer cells, the aforementioned microwave treatments and/or electroporation treatments can be applied to the treatment region, for example, to ablate the tumour.

According to an embodiment of the invention, there is provided an electrosurgical system comprising: an electrosurgical generator arranged to supply microwave energy and an electroporation signal; an electrosurgical instrument for inserting to a treatment region in biological tissue, the electrosurgical instrument comprising: a coaxial cable connected to the electrosurgical generator to receive the microwave energy and the electroporation signal, a rod-shaped radiating tip portion coupled to a distal end of the coaxial cable to receive the microwave energy and the electroporation signal, the radiating tip portion for radiating the microwave energy from its distal end into the treatment region and for establishing an electric field at its distal end using the electroporation signal to electroporate biological tissue in the treatment region, and a conduit for conveying biological tissue away from the treatment region; and a cytometer in fluid communication with the conduit to receive biological tissue, the cytometer being for detecting the presence of a first predetermined cell type in the received biological tissue. It is noted that detecting the presence of a particular cell type may include being able to sort or classify that particular cell type from one or more different cell types. For example, a cancerous cell may be sorted (e.g. separated) from healthy cells, so as to detect the cancerous cell. The healthy cells may be kept or discarded. It is noted that the classification "cancerous cell" may include one or more different cell types but all of which are cancerous. Also, the classification "healthy cell" may include one or more different cell types but all of which are non-cancerous.

The radiating tip portion may include: a proximal coaxial transmission line for receiving and conveying the microwave energy, the proximal coaxial transmission line including an inner conductor, an outer conductor and a dielectric material separating the inner conductor from the outer conductor; and a distal needle tip mounted at a distal end of the proximal coaxial transmission line, the distal needle tip comprising a rigid dielectric sleeve that extends the longitudinal direction from a distal end of the proximal coaxial transmission line, wherein the rod-shaped radiating tip portion has a diameter less than a diameter of the coaxial cable, wherein the rigid dielectric sleeve surrounds an elongate conductive element that is electrically connected to the inner conductor of the proximal coaxial transmission line and extends beyond a distal end of the outer conductor of the proximal coaxial transmission line, wherein the elongate conductive element is configured to operate as a half wavelength transformer for the microwave energy to thereby radiate the microwave energy from the distal needle tip into biological tissue, wherein the elongate conductive element terminates at an active electrode exposed on a distal end of distal needle tip, and wherein the active electrode is axially spaced from a return electrode that is electrically connected to the distal end of the outer conductor of the proximal coaxial transmission line, the active electrode and return electrode be configured to establish an electric field for electroporation of biological tissue at the distal needle tip.

The distal needle tip may be configured as a half wavelength transformer if its electrical length corresponds to a half wavelength of the microwave energy. An advantage of configuring the distal needle tip as a half wavelength transformer is to minimise reflections at the interface between components, e.g. between the coaxial cable and proximal coaxial transmission line, and between the proximal coaxial transmission line and the distal needle tip. A reflection coefficient at the latter interface is typically larger due to a larger variation in impedance. The half wavelength configuration minimises these reflections so that the dominant reflection coefficient becomes that of the interface between the proximal coaxial transmission line and the tissue. The impedance of the proximal coaxial transmission line may be selected to be identical or close to the expected tissue impedance to provides a good match at the frequency of the microwave energy.

As a result of the configuration of the radiating tip portion, the impedance of the coaxial transmission line may be 'seen' by the tissue rather than the (smaller) impedance of the distal needle tip structure. The physical length of the distal needle tip need not (indeed probably will not) correspond to a half wavelength of the microwave energy in free space, because the shape of distal needle tip and its interaction with the proximal coaxial transmission line can be selected to control the physical length of the distal needle tip whilst enabling it to operate electrically as a half wavelength transformer.

The coaxial cable may be configured to convey an electroporation signal which, when received by the rod-shaped radiating tip portion, establishes the electric field for electroporation of biological tissue at the distal needle tip. The active electrode may be disposed at a surface of the distal needle tip.

The electroporation waveform may comprise one or more high voltage energy pulses configured to open pores in cell membranes. The invention may be used in a scenario where a therapeutic agent is present at a treatment site, whereby opening pores in the cell membrane facilitates or enables the therapeutic agent to enter the cells. In other words, the invention may be used in conventional electroporation procedures. In an embodiment, the therapeutic agent (e.g. drug or local enchemotherapy) may be introduced to the treatment site via the conduit of the electrosurgical instrument.

Alternatively or additionally, the energy for electroporation may be configured to permanently open pores, thereby to cause irreversible disruption to the cell membrane causing the cells to die. In other words, the instrument can be used for irreversible electroporation (IRE).

The electroporation waveform may comprise one or more rapid high voltage pulses. Each pulse may have a pulse width in a range from 1 ns to 10 ms, preferably in the range from 1 ns to 100 µs, although the invention need not be limited to this range. Shorter duration pulses (e.g. equal to or less than 10 ns) may be preferred for reversible electroporation. For irreversible electroporation, longer duration pulses or more pulses may be used relative to reversible electroporation.

Preferably the rise time of each pulse is equal to or less than 90% of the pulse duration, more preferably equal to or less than 50% of the pulse duration, and most preferably equal to or less than 10% of the pulse duration. For the shorter pulses, the rise time may be of the order of 100 ps. In some examples, the electroporation waveform may be a radiofrequency (RF) or low frequency electromagnetic signal.

Each pulse may have an amplitude in the range 10 V to 10 kV, preferably in the range 1 kV to 10 kV. Each pulse may be positive pulse from a ground potential, or a sequence of alternating positive and negative pulses from a ground potential.

The electroporation waveform may be a single pulse or a plurality of pulses, e.g. a period train of pulses. The waveform may have a duty cycle equal to or less than 50%, e.g. in the range 0.5% to 50%.

In one example, pulse widths of the order of 200 ms delivered in a series of 10 to 100 pulses may be used for irreversible electroporation. In one example, the electroporation waveform may comprise 10 × 300 µs pulses of amplitude 1.5 kV delivered three times with around 1 minute between delivery. This waveform can cause cell apoptosis or death in hepatocellular carcinoma.

The electroporation waveform may be delivered during a treatment period that is selected depending on the desired effect. For example, the treatment period may be short, e.g. less than 1 second, or a few seconds, or around 1 minute. Alternatively the treatment period may be longer, e.g. up to an hour.

The coaxial cable may be a conventional low loss coaxial cable that is connectable at a proximal end to an electrosurgical generator. The coaxial cable may have a centre conductor separated from an outer conductor by a dielectric material. The coaxial cable may further include an outer protective sheath for insulating and protecting the cable. In some examples, the protective sheath may be made of or coated with a non-stick material to prevent tissue from sticking to the cable. The radiating tip portion is located at the distal end of the coaxial cable, and is connected to receive the EM energy conveyed along the coaxial cable.

The proximal coaxial transmission line may be connected to the distal end of coaxial cable. In particular, the inner conductor and outer conductor of the proximal coaxial transmission line may be electrically connected to the centre conductor and the outer conductor of the coaxial cable, respectively. The materials used in the proximal coaxial transmission line may be the same or different to those used in the coaxial cable. The materials used in the proximal coaxial transmission line may be selected to provide a desired flexibility and/or impedance of the proximal coaxial transmission line. For example, the dielectric material of the proximal coaxial transmission line may be selected to improve impedance matching with target tissue.

The dimensions of the components of the proximal coaxial transmission line may be chosen to provide it with an impedance that is identical or close to the impedance of the flexible coaxial cable (e.g. around 50 Ω). The inner conductor may be formed from a material with high conductivity, e.g. silver.

The radiating tip portion may be secured to the flexible coaxial cable by a collar or connector mounted over a junction therebetween. The collar may be electrically conductive, e.g. formed from brass. It may electrically connect the outer conductor with an outer conductor of the flexible coaxial cable.

An outer diameter of the radiating tip portion is smaller than an outer diameter of the coaxial cable. This may facilitate insertion of the radiating tip portion into target tissue, and improve the manoeuvrability of the radiating tip portion. This configuration may be particularly suited to treatment of tumours in the pancreas, as it may facilitate insertion of the radiating tip portion into the pancreas through the duodenum wall.

The radiating tip portion may include a non-stick coating (e.g. made of PTFE), to prevent tissue from sticking to it. The non-stick coating may be formed from Parylene C or Parylene D. The non-stick coating may be formed along the whole length of the radiating tip portion except for the active and return electrodes, which are exposed to facilitate efficient delivery of the electroporation signal into tissue. The non-stick coating may be applied only along a length corresponding to an active zone of ablation, e.g. along a region extending 2 cm back from the distal end (except for the active and return electrodes). When the needle is only partially coated, the needle may be less susceptible to a build-up of thermal energy, which can cause the needle to heat up.

In some embodiments, the return electrode may be formed by a distal portion of the outer conductor of the proximal coaxial transmission line. In this manner, the radiating tip portion may act as a bipolar electroporation electrosurgical instrument when it receives an electroporation waveform. By using the distal portion of the outer conductor as the return electrode, the electric field may be localised around the distal needle tip, so that electroporation may be performed in a region around the distal needle tip. The distal portion of the outer conductor may be located at the distal end of the proximal coaxial transmission line, adjacent to the distal needle tip. Where the outer conductor is formed from nitinol or some other flexible conductive material, the return electrode may include a coating formed on distal portion of the outer conductor of a material having a higher conductivity that the nitinol. The material may be silver, for example. To facilitate efficient delivery of the electroporation signal, the active and return electrodes may be polished, i.e. made as smooth as possible.

The elongate conductive element may radiate microwave energy along its length, to ablate tissue in a region located around the distal needle tip. In some cases, the elongate conductive element may be a distal portion of the inner conductor that extends into the distal needle tip.

The active electrode is electrically connected to the elongate conductive element. In this manner, the electroporation waveform may be delivered to the active electrode via the elongate conductive element. The active electrode may also serve to shape a microwave radiation profile of the radiating tip portion, e.g. to concentrate emission of microwave energy around the distal needle tip.

In some embodiments, the active electrode may be a conductive ring arranged concentrically with the elongate conductive element. In other words, a central axis of the conductive ring may be aligned with a longitudinal axis of elongate conductive element. This may serve to deliver the electroporation waveform to tissue symmetrically about the longitudinal axis. This may also serve to provide an axially symmetric microwave radiation profile.

The conductive ring may have a channel extending longitudinally therethrough, and a portion of the elongate conductive element may be contained within the channel. In this manner, the elongate conductor may be electrically connected to the active electrode inside the channel. A diameter of the channel may be dimensioned to substantially match an outer diameter of the elongate conductive element, so that the channel may form an interference fit around the elongate conductive element. This may serve to secure the active electrode relative to the elongate conductive element.

In some embodiments, the distal needle tip may comprise a tip element mounted at a distal end of the conductive ring. The tip element may be made of a dielectric material. The dielectric material of the tip element may be selected to improve impedance matching between the radiating tip portion and target tissue. A portion of the tip element may protrude within the channel, to hold the tip element in place relative to the channel.

A distal end of the tip element may be pointed (e.g. sharpened). This may facilitate insertion of the distal needle tip into target tissue. For example, this may facilitate insertion of the instrument through the duodenal or gastric wall into the pancreas.

The distal dielectric sleeve may have a bore formed therethrough for receiving the elongate conductive element. The distal dielectric sleeve may be made from a different material from the dielectric material in the proximal coaxial transmission line.

The distal dielectric sleeve may have a higher rigidity than the dielectric material of the proximal coaxial transmission line. Providing a higher rigidity to the distal dielectric sleeve may facilitate insertion of the distal needle tip into target tissue, whilst having a lower rigidity proximal coaxial transmission line may facilitate bending of the radiating tip portion. This may enable the instrument to be guided through narrow and winding passageways, whilst still enabling it to be inserted into target tissue. For example, the dielectric material of the proximal coaxial transmission line may be made of a flexible dielectric material (e.g. PTFE), and the distal dielectric sleeve may be made of e.g. a ceramic, polyether ether ketone (PEEK) or glass-filled PEEK. The tip element of the distal needle tip may be made of the same material as the distal dielectric sleeve.

In some embodiments, the distal dielectric sleeve may include zirconia. The inventors have found that zirconia provides a good rigidity for inserting the distal needle tip into tissue. Moreover, the inventors have found that using a zirconia distal dielectric sleeve may provide good impedance matching with target tissue.

In some embodiments, a distal portion of the outer conductor may overlay a proximal portion of the distal dielectric sleeve. In other words, the proximal portion of the distal dielectric sleeve may be contained within the distal portion of the outer conductor. This may serve to strengthen the connection between the distal needle tip and the proximal coaxial transmission line.

The length of the radiating tip portion where the distal portion of the outer conductor overlays the proximal portion of the distal needle tip may form an intermediate coaxial transmission line between the proximal transmission line and the distal needle tip. The intermediate coaxial transmission line may have a higher dielectric constant than the proximal coaxial transmission line to allow for a smaller physical length whilst getting the required electrical length (half wave). At microwave frequencies, a distal portion of the distal needle tip may act as an open-ended loaded monopole connected to the intermediate coaxial transmission line. The distal needle tip may also be considered as a single structure which ends in an open-ended co-axial monopole to shape the ablation zone.

In some embodiments, the distal dielectric sleeve may formed by a pair of cooperating parts, each one of the cooperating parts having a longitudinal groove formed in a surface thereof for receiving the elongate conductor. Such a structure of the distal dielectric sleeve may facilitate assembly of the radiating tip portion. When the cooperating parts are assembled to form the distal dielectric sleeve, the grooves in the cooperating parts may form a bore in which the elongate conductor is received. The cooperating parts may be secured together using an adhesive.

In some embodiments, the outer conductor of the proximal coaxial transmission line may be formed from nitinol. For example, the outer conductor may be formed of a nitinol tube. The inventors have found that nitinol exhibits a longitudinal rigidity sufficient to transmit a force capable of penetrating the duodenum wall. Additionally, the flexibility of nitinol may facilitate bending of the radiating tip portion, so that the instrument may be guided through narrow bending passageways. Forming the outer conductor of nitinol may thus facilitate use of the instrument for treatment of tumours in the pancreas.

A conductive outer layer may be formed on an outer surface of the outer conductor, the conductive outer layer having a higher conductivity than nitinol. The conductive outer layer may serve to reduce losses of microwave energy in the radiating tip portion, to improve efficiency of microwave energy delivery to the distal needle tip. A thickness of the conductive outer layer may be smaller than a thickness of the nitinol, to minimise any impact of the conductive outer layer on flexibility of the radiating tip portion.

The radiating tip portion may have a length equal to or greater than 30 mm and preferably 40mm, but could be as long as 100 mm. This length may enable access to treatment regions at all locations within the pancreas. The radiating tip portion may have a maximum outer diameter equal to or less than 1.2 mm. For example the maximum outer diameter may be similar to or the same as 19G (1.067mm) or 22G (0.7176mm). This may reduce or minimise the penetration hole caused by insertion of the instrument, so as not to cause an undue delay in healing. Minimising the size of the penetration hole may also avoid the undesirable situation of it healing open and causing a fistula or unwanted channel between the GI tract and the body cavity.

In some embodiments, the inner conductor may extend from a distal end of the flexible coaxial cable, the inner conductor being electrically connected to a centre conductor of the flexible coaxial cable, and the inner conductor may have a diameter that is less than the diameter of the centre conductor of the flexible coaxial cable. This may improve the flexibility of the radiating tip portion. For example, the diameter of the inner conductor may be 0.2mm to 0.4mm. The diameter of the inner conductor may take into account that the dominant parameter that determines loss (and heating) along the radiating tip portion is the conductor loss, which is a function of the diameter of the inner conductor. Other relevant parameters are the dielectric constants of the distal dielectric sleeve and dielectric material of the proximal coaxial transmission line, and the diameter and material used for the outer conductor.

In one embodiment, the conduit may be integrated with the radiating tip portion. For example, the conduit may be a hollow channel or bore in the inner conductor and the elongate conductive element. Also, where the radiating tip portion includes a tip element, the conduit may be a hollow channel or bore in the tip element. This arrangement offers the advantage of a compact system. It is achievable because the skin depth of the microwave energy proposed herein in a good conductor is small enough for the inner conductor and the elongate conductive element to be hollow without substantially affecting the energy conveyed.

In an embodiment, the conduit includes at least one pipe connected to the inner conductor for conveying the biological tissue away from the bore.

In one embodiment, the conduit extends along the axis of the proximal coaxial transmission line and includes an outlet on the axis. In this embodiment, the coaxial cable may be side-fed rather than end-fed, i.e. a connector may be arranged at an angle to the proximal coaxial transmission line, i.e. at 90° to the length of the structure. As such, the microwave energy and electroporation signal are connected to the radiating tip portion at an angle, but biological tissue is extracted in-line with a longitudinal axis of the electrosurgical instrument. In an alternative embodiment, the microwave energy and the electroporation signal are fed into the radiating tip portion along the axis of the proximal coaxial transmission line (i.e. in-line) and the biological tissue is extracted using at least one pipe that is angled to the axis of the proximal coaxial transmission line (e.g. at 90° to the length of the structure). As such, the microwave energy and electroporation signal are connected to the radiating tip portion in-line with the longitudinal axis of the electrosurgical instrument, but biological tissue is extracted at an angle.

The electrosurgical system may further include a surgical scoping device (e.g. an endoscope) having a flexible insertion cord for insertion into a patient's body, wherein the flexible insertion cord has an instrument channel running along its length, and wherein the electrosurgical instrument is dimensioned to fit within the instrument channel.

The term "surgical scoping device" may be used herein to mean any surgical device provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube. The instrument channel may have a diameter suitable for receiving invasive surgical tools. The diameter of the instrument channel may be 5 mm or less. In embodiments of the invention, the surgical scoping device may be an ultrasound-enabled endoscope.

The electrosurgical system may include a controller for controlling the microwave energy and the electroporation signal supplied by the electrosurgical generator. For instance, the controller may be used to set a power or frequency of the microwave energy. Also, the controller may be used to set a pulse width, pulse duty cycle or pulse amplitude of the electroporation signal. In this way, the controller provides a mechanism for controlling the effects of the microwave energy, for example to measure tissue or to ablate tissue, and of the electroporation signal, for example, to perform reversible electroporation or irreversible electroporation.

In use, microwave power delivered by the electrosurgical system may be reflected by different amounts due to the different impedance values for different types of biological tissue; this corresponds to an impedance mismatch between the radiating tip portion and the contact tissue. Such reflections may be taken into account when selecting the output power level of the microwave energy from the generator. Alternatively, the system may monitor and adjust the power delivered to the electrosurgical instrument. For example, the system may include a detector for detecting microwave power reflected back from the treatment region and the controller may adjust a controllable power level of microwave radiation based on changes in the detected reflected microwave power.

The electrosurgical instrument may have the ability to perform measurements of tissue. The ability to measure dielectric properties of the tissue (the measured information) may offer significant advantage in terms of locating cancerous tissue the first time the electrosurgical instrument is inserted into the region of tissue where it is suspected that a tumour is present, i.e. there may be no need to take a number of tissue samples. Also, the ability to measure tissue properties in this manner may reduce the risk of false negatives occurring. Specifically, a change in reflected power, e.g. a change in the magnitude of a microwave signal travelling back from the interface between the electrosurgical instrument and biological tissue, may indicate a change in the type of material present at the distal end of the electrosurgical instrument. The controller may be arranged to recognise certain expected changes, e.g. from healthy tissue to cancerous tissue. In an embodiment, the controller may notify a user of the system when a certain tissue type is detected (e.g. via a user interface).

The detector may also be arranged to detect forward power delivered to the electrosurgical instrument. The controller may thus be able to determine the amount of power being delivered to the biological tissue. The controller may be arranged to adjust the controllable power level of microwave radiation based on the detected forward and reflected microwave power to deliver microwave energy according to a predetermined energy delivery profile. The controller may be arranged to select the predetermined energy delivery profile from a plurality of predetermined profiles based on changes in the detected reflected microwave power.

Each predetermined energy delivery profile may be linked with a tissue type. For example, an energy delivery profile for blood may be arranged to ensure delivery of enough power to cause a rise in temperature that would seal a broken blood vessel. Also, an energy delivery profile for cancerous tissue may be arranged to ensure delivery of enough power to ablate the tissue.

The controller may be arranged to measure the magnitude (and/or phase) of the impedance of the biological tissue at the distal end of the electrosurgical instrument and to select a predetermined energy delivery profile according to the measured impedance.

To ensure accurate detection, the system may be arranged to isolate the reflected power from the forward power. For example, the system may include a circulator connected between the generator, instrument and detector, wherein a forward path for microwave energy from the generator passes from a first port to a second port of the circulator, a reflected path for microwave energy from the instrument passes from the second port to a third port of the circulator, and the detector includes a first directional coupler connected to couple power output from the third port of the circulator.

To detect forward power, the detector may include a second directional coupler connected to couple power input to the first port of the circulator.

To improve isolation between the forward and reflected paths, one or more additional circulators may be connected between the second directional coupler and the circulator. This invention is not limited to the use of one or more circulators to provide the necessary isolation between the forward going and reflected signals, i.e. a directional coupler with a high value of directivity, e.g. a waveguide coupler, may be used.

The microwave energy source of the electrosurgical generator may have an adjustable output frequency. For example, there may be more than one oscillator in the source, each oscillator being selectively connectable to an amplifier. Alternatively, the source may include a variable frequency generator. The frequency may be selected before use, e.g. depending on the tissue to be treated or the size of the treatment region. The controller may be arranged to adjust the frequency in use, e.g. based on changes in the reflected microwave power.

The system may include an impedance matching mechanism arranged to match the impedance of the radiating tip portion in the electrosurgical instrument with the biological tissue at the distal end of the instrument during a surgical procedure (e.g. tunnelling). The impedance adjustment and/or energy profile adjustment based on variations in impedance presented to the radiating tip portion may be used to ensure that a track of ablation with a constant diameter is created during the tunnelling procedure.

The cytometer (aka cell sorter) may be a dielectrophoresis cell sorter, in that, the cytometer uses electromagnetic fields to selectively electro-manipulate cells with dielectrophoresis (DEP) forces such that the cells are dynamically sorted into different physical locations or bins depending on their susceptibility to the electromagnetic field. That is, exposing a first predetermined cell type (e.g. a cancerous cell) to an particular electromagnetic field may force that cell to adopt a first trajectory into a first physical location (e.g. well or bin), whereas exposing other cells (e.g. a healthy cell) to the same electromagnetic field may force those cells to adopt a second trajectory into a second physical location. In this way, cancerous cells and non-cancerous (e.g. healthy) cells are sorted or classified into groups, with each group being positioned at a different location. In this way, identifying the presence of cells at a particular location (e.g. the first physical location) provides a mechanism for determining or detecting the presence of the first predetermined cell type (e.g. cancerous cells).

Also, the cytometer may be part of a cell-identification assembly (or module) which functions to extract cells from a treatment site, prepare the extracted cells for cell sorting and then uses the cytometer to identify the presence of a first predetermined cell type. For instance, the cell identification assembly may include a suction pump in fluid communication with (e.g. connected to) the conduit so as to extract biological tissue from a treatment region at a distal end of the electrosurgical instrument. Also, the cell identification assembly may include a sample generator which suspends cells from the extracted biological tissue in a fluid in order to generate a sample or cytometer sample. Next the sample is provided to the cytometer such that cell sorting can be performed on cells of the extracted biological tissue in order to determine the presence of one or more particular cell types. In an embodiment, the cytometer is configured to identify cancer stem cells. In another embodiment, the cytometer is configured to distinguish between healthy cells and cancerous cells, or cancer stem cells and cancerous cells.

The system may further include a fluid injecting mechanism in fluid communication with the conduit, wherein the fluid injecting mechanism is operable to inject fluid (e.g. drugs or local chemotherapy) into the treatment region. For example, the fluid injecting mechanism may include a tank (or compartment or vessel) in fluid communication with the conduit, and a suction pump for injecting fluid from the tank into the treatment region at a distal end of the electrosurgical instrument. The fluid injecting mechanism may share at least some of the components of the cell identification assembly (e.g. a suction pump, or vessel). For example, a fluid line may extend away from the electrosurgical instrument and branch into two separate paths at a junction. The junction may include one or more valves which are controllable (e.g. by a controller) to select between a first path, from the electrosurgical instrument to the cytometer (e.g. for cell detection), and a second path, from the fluid injecting mechanism to the electrosurgical instrument (e.g. for fluid injection).

Herein, the term "inner" means radially closer to the centre (e.g. axis) of the instrument channel and/or coaxial cable. The term "outer" means radially further from the centre (axis) of the instrument channel and/or coaxial cable.

The term "conductive" is used herein to mean electrically conductive, unless the context dictates otherwise.

Herein, the terms "proximal" and "distal" refer to the ends of the elongate electrosurgical instrument. In use, the proximal end is closer to a generator for providing the RF and/or microwave energy, whereas the distal end is further from the generator.

In this specification "microwave" may be used broadly to indicate a frequency range of 400 MHz to 100 GHz, but preferably the range 1 GHz to 60 GHz. Preferred spot frequencies for microwave EM energy include: 915 MHz, 2.45 GHz, 3.3 GHz, 5.8 GHz, 10 GHz, 14.5 GHz and 24 GHz. 5.8 GHz may be preferred. The device may deliver energy at more than one of these microwave frequencies.

The term "radiofrequency" or "RF" may be used to indicate a frequency between 300 kHz and 400 MHz. The term "low frequency" or "LF" may mean a frequency in the range 30 kHz to 300 kHz.

### BRIEF DESCRIPTION OF THE DRAWINGS

Embodiments of the invention are discussed below with reference to the accompanying drawings, in which:
Fig. 1 is a schematic diagram of an electrosurgical system that is an embodiment of the invention;
Fig. 2(a) is a is a block diagram showing part of an electrosurgical system that is an embodiment of the invention;
Fig. 2(b) shows a schematic diagram of the cytometer of Fig. 2(a).
Fig. 3 is a schematic cross-sectional side view of an electrosurgical instrument according to an embodiment of the invention;
Fig. 4(a) is a schematic cross-sectional side view of a distal end of the electrosurgical instrument of Fig. 3;
Fig. 4(b) is a schematic cross-sectional side view of a proximal end of the electrosurgical instrument of Fig. 3;
Fig. 5 shows schematic diagrams of an active electrode that may be used in an embodiment of the invention;
Fig. 6 shows schematic diagrams of a tip element that may be used in an embodiment of the invention;
Fig. 7 shows schematic diagrams of a part of a distal dielectric sleeve that may be used in an embodiment of the invention;
Fig. 8 is a schematic perspective view of another tip element that can be used in the invention;
Fig. 9 is a cross-sectional view of a distal tip portion of an instrument that includes the tip element of Fig. 8; and
Fig. 10 shows a schematic cross-sectional side view of an alternative proximal end of the electrosurgical instrument of Fig. 3.

### DETAILED DESCRIPTION; FURTHER OPTIONS AND PREFERENCES

In this description the term ablation may refer to the ablation of a region of cancerous tissue, for example a tumour, or for sealing a track or channel made as the electrosurgical instrument passes through layers of healthy tissue. The latter will generally require lower levels of power and the track ablation may be performed with dynamic energy matching to the tissue impedance seen en route to ensure that controlled amounts of energy is launched into the various tissue types as electrosurgical instrument traverses back through the tissue. However this invention need not be limited to performing controlled ablation with dynamic impedance matching being in place.

In an embodiment, an electrosurgical instrument substantially as described in GB patent application no. 1819683.2 is used. This electrosurgical instrument is modified such that it can perform as a trifunctional antenna substantially as described in PCT/GB2007/003842 or as an electrosurgical instrument substantially as described in PCT/GB2010/001858 - for example, the electrosurgical instrument is modified to include a channel for extracting biopsy tissue (e.g. fluid or cells). Furthermore, the combination is then modified to include a cell identification assembly or cytometer (aka cell sorter) so that cells obtained via biopsy can be classified, for example, between either healthy and cancerous cells, or cancerous cells and cancer stem cells. In use, the electrosurgical instrument can be used to perform electroporation on cells in order to sensitise them i.e. to open up their cell membrane (or pores) to make them more sensitive to microwave energy. Also, drugs (e.g. local chemotherapy) can be delivered to the sensitised cells, for example, via the biopsy channel, and then microwave energy can be used to activate the chemotherapy. Additionally, irreversible electroporation can be performed on cells to ablate them. Further, ablation can performed using microwave energy.

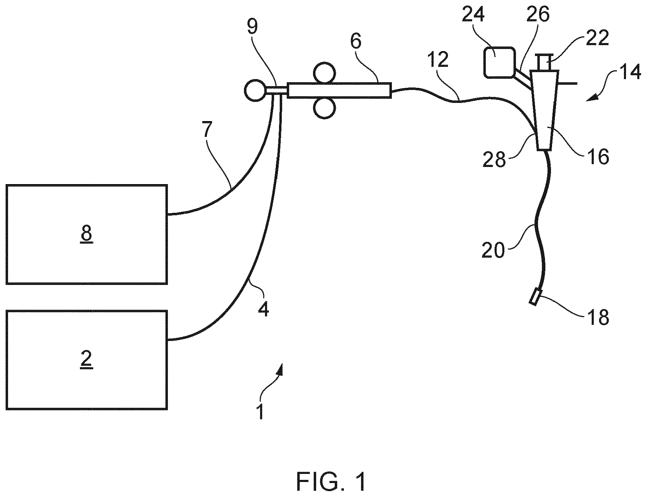

Fig. 1 is a schematic diagram of an electrosurgical ablation apparatus 1 that is capable of supplying microwave energy and energy for electroporation to the distal end of an invasive electrosurgical instrument. The system 1 comprises a generator 2 for controllably supplying microwave energy and energy for electroporation. Energy for electroporation may comprise pulsed or sinusoidal (e.g. continuous wave electromagnetic wave) energy in the radiofrequency (RF) or low frequency (LF) bands.

A suitable generator for this purpose is described in WO 2012/076844. The generator may be arranged to monitor reflected signals received back from the instrument in order to determine an appropriate power level for delivery. For example, the generator may be arranged to calculate an impedance seen at the distal end of the instrument in order to determine an optimal delivery power level.

The generator 2 is connected to an interface joint 6 by an interface cable 4. In the example shown, the interface joint 6 is also connected via a fluid flow line 7 to a fluid system 8. In some examples, the fluid system 8 includes a collection tank (or vessel), and a pump. The collection tank may be used to collect biopsy tissue (fluid or cells) from a treatment site proximal to a distal end assembly (see below), and the pump may be used to suck the tissue sample into the tank. Additionally, the fluid system 8 may include a mechanism for introducing fluid (e.g. drugs or local chemotherapy) into the treatment site. In any case, the fluid flow line 7 conveys fluid between the interface joint 6 and the fluid system 8. The fluid system 8 also includes a cytometer or cell sorter for sorting cells of the biopsy tissue in order to identify the presence of one or more particular cell types in the biopsy tissue. For example, the cytometer may be configured to classify cells as one or more of the following: healthy cells, cancer cells, cancel stem cells. In an embodiment, the cytometer is operable to differentiate between either healthy cells and cancerous cells, or cancerous cells and cancer stem cells.

If needed, the interface joint 6 can house an instrument control mechanism that is operable by sliding a trigger, e.g. to control longitudinal (back and forth) movement of one or more control wires or push rods (not shown). If there is a plurality of control wires, there may be multiple sliding triggers on the interface joint to provide full control. The function of the interface joint 6 is to combine the inputs from the generator 2, fluid system 8 and instrument control mechanism into a single flexible shaft 12, which extends from the distal end of the interface joint 6.

The flexible shaft 12 is insertable through the entire length of an instrument (working) channel of a surgical scoping device 14, which in embodiments of the present invention may comprise an endoscopic ultrasound device.

The surgical scoping device 14 comprises a body 16 having a number of input ports and an output port from which an instrument cord 20 extends. The instrument cord 20 comprises an outer jacket which surrounds a plurality of lumens. The plurality of lumens convey various things from the body 16 to a distal end of the instrument cord 20. One of the plurality of lumens is an instrument channel for receiving the flexible shaft 12. Other lumens may include a channel for conveying optical radiation, e.g. to provide illumination at the distal end or to gather images from the distal end, and an ultrasound signal channel for conveying an ultrasound signal. The body 16 may include an eye piece 22 for viewing the distal end.

An endoscopic ultrasound device typically includes an ultrasound transducer on a distal tip of the instrument cord, beyond an exit aperture of the ultrasound signal channel. Signals from the ultrasound transducer may be conveyed by a suitable cable 26 back along the instrument cord to a processor 24, which can generate images in a known manner. The instrument channel may be shaped within the instrument cord to direct an instrument exiting the instrument channel through the field of view of the ultrasound system, to provide information about the location of the instrument at the target site.

The flexible shaft 12 has a distal assembly 18 (not drawn to scale in Fig. 1) that is shaped to pass through the instrument channel of the surgical scoping device 14 and protrude (e.g. inside the patient) at the distal end of the instrument cord.

The structure of the distal assembly 18 discussed below may be particularly designed for use with an endoscopic ultrasound (EUS) device, whereby the maximum outer diameter of the distal end assembly 18 is equal to or less than 2.0 mm, e.g. less than 1.9 mm (and more preferably less than 1.5 mm) and the length of the flexible shaft 12 can be equal to or greater than 1.2 m. In an embodiment, the maximum outer diameter of the distal end assembly 18 is about 19G (1.067mm) or 22G (0.7176mm).

The body 16 includes a power input port 28 for connecting to the flexible shaft 12. As explained below, a proximal portion of the flexible shaft 12 may comprise a conventional coaxial cable capable of conveying the microwave energy and electroporation energy from the generator 2 to the distal assembly 18.

As discussed above, it is desirable to be able to control the position of at least the distal end of the instrument cord 20. The body 16 may include a control actuator that is mechanically coupled to the distal end of the instrument cord 20 by one or more control wires (not shown), which extend through the instrument cord 20. The control wires may travel within the instrument channel or within their own dedicated channels. The control actuator may be a lever or rotatable knob, or any other known catheter manipulation device. The manipulation of the instrument cord 20 may be software-assisted, e.g. using a virtual three-dimensional map assembled from computer tomography (CT) images.

In general terms, one embodiment of the distal assembly 18 (aka needle antenna or antenna structure) comprises any suitable antenna structure that enables microwave energy to be transferred in the forward and reverse direction to enable the measurement of dielectric information, and to cause controlled tissue ablation or tissue measurement, whilst allowing tissue samples (fluid or cells) to be extracted without upsetting the environment set-up to allow microwave signals to propagate for the purpose of making a dielectric measurement or for the purpose of introducing a high enough level of microwave energy into biological tissue to cause controlled tissue ablation. Additionally, the antenna structure must be cable of establishing an electric field using an electroporation signal in order to perform electroporation (e.g. reversible or irreversible electroporation) of tissue (e.g. cells).

The invention makes use of the fact that the centre conductor within the antenna is around 0.3-0.5 mm in diameter, but a wall thickness of about 0.01 mm only is required to enable almost all of the microwave energy to flow, or to be transported, along an appropriate conductive material when the frequency of operation is 14.5 GHz. Thus, in theory the centre of the centre conductor can be removed to leave a bore (or conduit) having a diameter of about 0.2-0.4 mm available as a channel that can be used to inject or extract fluid to or from a treatment site, for example, to remove fluid from a cyst or cells within a solid mass. It is worthwhile noting that this channel could also be used to transport other liquids and/or solids in and out of the needle antenna. For example fluid, drugs (e.g. local chemotherapy), imaging or contrast media for specific tissue marking and/or identification.

In this description, an antenna structure and system is described that has the potential to perform the following functions:
- measure dielectric information to determine the type, state and location of healthy and cancerous tissue,
- perform a needle biopsy with confidence that the tip of the needle is located inside the centre of the tumour, or other biological tissue that may require treatment,
- perform a needle biopsy and use cell sorting or cytometry on the biopsy tissue to identify the presence of particular cell types (e.g. cancerous cells),
- controllably ablate (via microwave energy and via irreversible electroporation) the tumour or other unhealthy tissue structures and a small region of healthy tissue (a safe margin),
- controllably sensitise cells via reversible electroporation to open up their cell membranes (or pores) and make them more susceptible to microwave ablation,
- deliver drugs (e.g. local chemotherapy) into sensitised cells and then active the drugs using microwave energy (e.g. via heating without ablating),
- take further needle biopsies during and after the treatment process, and
- controllably ablate the channel (via microwave energy or via irreversible electroporation) made by the needle antenna during needle withdrawal to prevent seeding.

The combined procedure involving tissue measurement, tissue biopsy, and tissue ablation can allow cancerous tissue (fluid or cells) to be located during a first attempt, and the risk of dragging cancerous cells back through the channel can be mitigated due to the fact that the needle channel (or track) is subjected to controlled ablation, thus causing the death of any cancerous cells that may be present at or around the distal tip of the needle antenna.

It should be noted that this device can be used to perform any combination of the above listed functions. For example, it could be used to: (i) measure dielectric information to determine the type, state and location of healthy and cancerous tissue; (ii) on locating cancerous tissue, perform a needle biopsy with confidence that the tip of the needle is located inside the centre of the tumour; (iii) use cell sorting or cytometry on the biopsy tissue to identify the presence of a particular cell type (e.g. cancer stem cells); (iv) controllably sensitise cells via reversible electroporation to open up their cell membranes (or pores) and make them more susceptible to microwave ablation; (v) deliver drugs (e.g. local chemotherapy) into sensitised cells and then active the drugs using microwave energy (e.g. via heating without ablating); and (vi) controllably ablate the channel (via microwave energy or via irreversible electroporation) made by the needle antenna during needle withdrawal to prevent seeding.

It may also be desirable to use the current invention to deposit materials (e.g. chemotherapy) into the biological system instead of or in addition to removing tissue from the biological system. In this mode of operation, the tissue measurement and characterisation feature may be used to identify the region of the body where a material (solid or liquid) is required to be located with a high degree of accuracy, and the material may be deposited at the exact desired location (features associated with the use of the low power microwave frequency transceiver facilitates this). This aspect of the current invention may be particularly useful for depositing a particular drug or a radioactive dye into the body for example. This idea may be used with brachytherapy. The ability to target the exact location where a drug is to be delivered may offer significant advantage in terms of minimising the concentration and amount of drug required.

It should also be noted that the centre tube may be used to suck out or remove ablated tissue in order to increase the zone of ablation. This may be of particular use where the ablated tissue has become charred. Once the tissue has been removed the ablation process may commence again and the process repeated a number of times.

This invention is not limited to removing fluid or cells associated with cancerous tumours; the needle antenna may be used to remove other tissue from sensitive regions of the body where it is required to accurately locate the biopsy tissue inside target tissue. In these applications, the invention may be operated in measurement mode only.

A feature of the current invention may be to pump water or saline through the biopsy channel during ablation to keep the needle antenna as cool as possible. It may be advantageous to use this feature in applications where it is desirable to treat large lesions. In this instance, it may be required for the level of microwave power to be increased from that used when operating in the treatment mode under normal conditions, for example, where spherical tumours of diameter greater than 2 cm are to be treated, or where it is required to deliver power over longer durations of time. For example it may be required to generate up to 100 W of continuous wave (CW) power for ten minutes in order to treat a spherical lesion of, for example, 10 cm in diameter.

Alternatively, the biopsy may be used to introduce a material (e.g. lossy biocompatible material) which can augment the ablation effect, e.g. increase the ablation volume that is achievable with the apparatus. The presence of the material within the needle may not affect the generated microwave field because the microwave energy only flows in the outer section of the inner conductor.

In one embodiment, the biopsy channel may be used to suck necrosed or charred tissue from the needle tip during ablation. This may be particularly beneficial where dynamic impedance matching is implemented because it removes the charred tissue that the needle would otherwise have to be matched with. Typically charred tissue presents a load that is very different from that which the needle may be designed to match with in the absence of a tuner.

### Biopsy apparatus

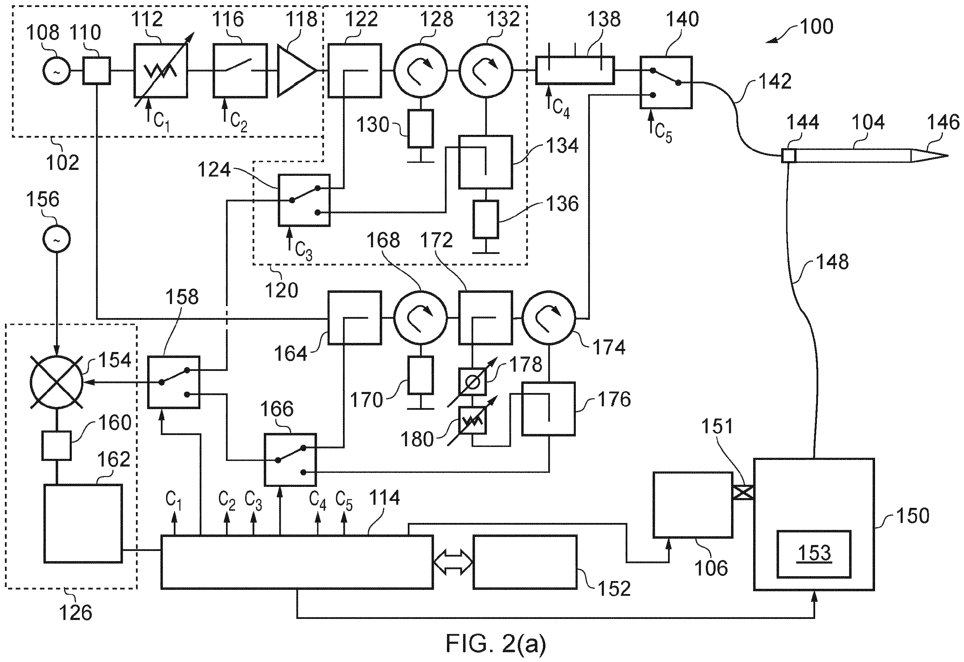

Fig. 2(a) shows a block diagram of part of the overall system. This configuration enables (i) biopsy, (ii) tissue ablation via microwave energy, and (iii) tissue measurement, modes of operation to be performed using a electrosurgical instrument 104, which may include the distal end assembly and needle antenna mentioned above. It is to be understood that, as mentioned above, the electrosurgical instrument 104 is also configured to perform electroporation, however, for clarity, the apparatus required to enable the electroporation capabilities is not shown in Fig. 2(a).

The apparatus 100 comprises a first (treatment) channel having a microwave energy power source 102 connected to deliver microwave energy to the electrosurgical instrument 104. The electrosurgical instrument 104 includes a conduit (not shown) for collecting biopsy tissue (fluid or cells) from a treatment site using suction provided by suction pump 106.

The source 102 comprises an oscillator 108, e.g. a voltage controlled oscillator or dielectric resonant oscillator, arranged to output a signal at a stable frequency, e.g. 14.5 GHz. The oscillator 108 may be connected to a stable crystal reference in a phased locked loop configuration (not shown) to keep its frequency steady. The output of the oscillator is connected to the input port of a power splitter 110 (e.g. 3 dB splitter), which separates the output signal between a treatment channel and a measurement channel (discussed below). The measurement channel may not be needed (i.e. is optional), so the splitter 110 may be optional. The output from the splitter 110 on the treatment channel is received by a variable attenuator 112, whose function is to vary the amplitude of the signal under the control of control signal C1 from controller 114 in order to adjustably control the overall output power level of the treatment channel. The output from the variable attenuator 112 is received by a switch 116 (e.g. a PIN diode switch), whose function is to modulate the signal under the control of control signal C2 from the controller 114 in order to enable pulsed operation (or another modulation format, i.e. a triangular waveform or a ramp falling abruptly to zero once maximum value has been reached). Other possible shapes include: a continuous wave or a square-wave pulsed signal, a Gaussian shape profile, or a rounded profile. The output from the switch 116 is received by a power amplifier 118 (e.g. an array of MMIC amplifiers), whose function is to amplify the power level of the signal to a level suitable for treatment. A particular embodiment of power amplifier 118 is a Triquint TGA4521-EPU MMIC, whose output is connected in cascade to the input of a higher power Triquint TGA4046-EPU MMIC. The TGA4521-EPU device is capable of producing a gain of 15 dB and a power level of 23 dBm (200 mW) when driven into saturation using an appropriate drive signal at a frequency of up to 47 GHz, and the TGA4046-EPU device is capable of producing a gain of 16dB and a power level of 33 dBm (2 W) when driven into saturation using an appropriate drive signal at a frequency of up to 46 GHz. In this embodiment, the system may be driven using source oscillator 108 outputting a frequency of 46 GHz and an output power of 2 dBm to enable 2 W of power to be produced at the output of the second MMIC connected in the cascade arrangement. Source oscillator 108 may be a device available through Castle Microwave Ltd, part number: OFD-KF460105-01, which is a dielectric resonator oscillator that is capable of producing an output power level of up to 5 dBm, has a mechanical tuning range of ± 25MHz, a frequency stability of ± 4 ppm/degree C, and phase noise of -95 dBc/Hz at 100 kHz offset.

As explained above, control of the power input to the amplifier 118 using the variable attenuator 112 enables control of the output power level.

The output power level may be dynamically controlled based on information from a detector 120 that is connected on the treatment channel between the source 102 and the electrosurgical instrument 104. In this embodiment, the detector 120 is arranged to detect both forward signals from the source 102 to the electrosurgical instrument 104 and reflected signals travelling back from the electrosurgical instrument 104. In other embodiments the detector may only detect reflected signals. In yet further embodiments the detector may be omitted altogether.

The detector 120 comprises a forward directional coupler 122 connected to couple power from the output of the amplifier 118. The coupled port of the coupler 122 is connected to a switch 124, whose function is to select either the forward coupled or reflected coupled power under the control of control signal C3 from controller 114 to be conveyed for measurement by a heterodyne detector 126. The output of the forward directional coupler 122 on the treatment channel is received by the first port of a first circulator 128, whose function is to isolate the reflected signals travelling back from the electrosurgical instrument 104 from the source 102. Forward signals on the treatment channel travel from the first port of the first circulator 128 to its second port, where they are output. Any reflected signals received at the second port of the first circulator 128 travel to the third port and are output into a power dump load 130. The output from the second port of the first circulator 128 is received by the first port of a second circulator 132, whose function is to convey the reflected signal towards a reflected directional coupler whilst isolating the reflected signal from the forward signal. Forward signals on the treatment channel travel from the first port of the second circulator 132 to its second port, where they are output. Reflected signals from the electrosurgical instrument 104 are received at the second port of the second circulator 132, from where they travel to the third port and are output. The output of the third port of the second circulator 132 is received by a reflected directional coupler 134, whose function is to couple power from the reflected signal. After passing through the coupler 134, the reflected signal is absorbed in a power dump load 136. The coupled port of the reflected power coupler 134 is connected to the switch 124 to be conveyed to the heterodyne detector 126 when selected. It is advantageous to use two circulators in this configuration, but this invention is not limited to the use of two, i.e. one, three, or more may be used.

The output from the detector 120 on the treatment channel is received by an impedance tuning mechanism 138, whose function is to match the impedance of the components on the treatment channel with the impedance of the electrosurgical instrument 104 when it is in tissue to facilitate efficient power transfer into tissue. The impedance tuning mechanism 138 may be optional. In this embodiment, the impedance tuning mechanism 138 comprises a cavity with three stubs insertable therein under the control of control signal C4 from controller 114. The impedance tuning mechanism 138 may be as described in WO 2005/115235. The impedance tuning mechanism may be operational only during insertion (tunnelling) of the electrosurgical instrument as discussed below. The impedance adjusting mechanism need not be limited to this configuration, i.e. it could comprise a single or plurality of power varactor or power PIN diodes connected to a microstrip or other transmission line between the output of the power generator and the antenna, or a variable (or adjustable) length of microstrip (or stripline) configured as a variable tuning stub that can also be moved along a constant impedance microstrip or other transmission line between the output of the generator and the antenna. All tuning positions may be achieved by a change in length of the variable stub and its movement along the microstrip or coaxial line may be between limited to up to half the loaded wavelength at the frequency of interest.

The output from the impedance tuning mechanism 138 is received by a switch 140, whose function is to select either a treatment channel signal or a measurement channel signal for input to the electrosurgical instrument 104 under the control of control signal C5 from controller 114. This switch may be a waveguide switch, a power varactor/PIN diode switch, a coaxial switch, or the like.

The output signal from the switch 140 is conveyed to the electrosurgical instrument 104 by a flexible transmission cable 142 (e.g. coaxial cable) that is terminated in a connector 144 on the electrosurgical instrument 104. The cable 142 may form part of the electrosurgical instrument 104. The connector 144 transfers the signal to an antenna (not shown) which includes an aerial (or radiating tip portion) 146 arranged to emit a microwave radiation field from the distal end of the electrosurgical instrument 104. The frequency of the microwave radiation and the power level of the signal sent to the electrosurgical instrument are selected such that the microwave radiation field adopts configurations in tissue that enable various capabilities of the system, for example, tissue ablation, tissue measurement, or activation of drugs (e.g. chemotherapy) contained within the tissue.

A conduit or bore (not shown) in the electrosurgical instrument 104 includes one or more openings at the distal end of the electrosurgical instrument 104. The proximal end of the conduit is connected via a transport pipe 148 to a collection tank or vessel 150, which is used to collect biopsy tissue (fluid or cells) present at a distal end of the electrosurgical instrument 104. A pump 106 is used to suck the tissue sample along the conduit within electrosurgical instrument 104 (not shown here), and suck the tissue through transport pipe 148 into tank 150. It must be ensured that there are no leaks in the system. A valve 151 is used to ensure that tissue cannot be directed into pump 106. Controller 114 is used to control the operation of pump 106. It may be desirable to attach fluid level monitors or sensors (not shown) inside tissue vessel 150 to monitor the level of tissue inside the vessel; controller 114 may be used to process signals from level monitors or sensors and this information may be displayed using user interface 152. Controller 114 may also be used to control the operation of a valve (not shown), which is used to empty vessel 150. The operation of this valve may be based on information obtained from the level sensors.

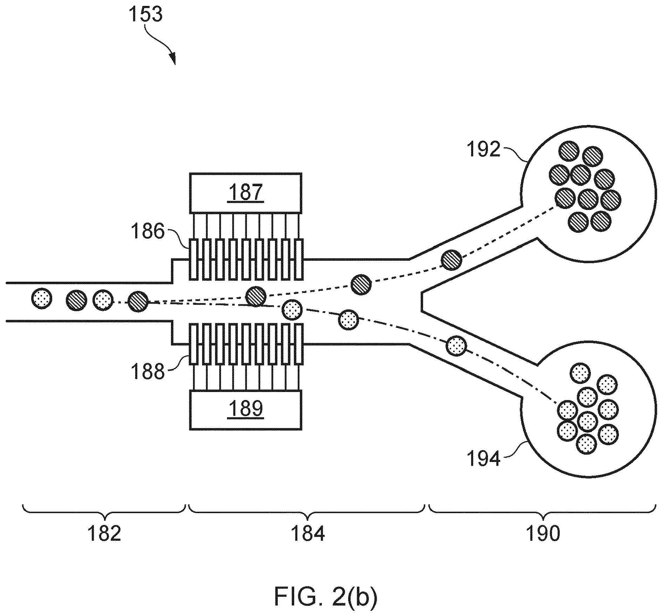

The vessel 150 includes a cytometer (or cell sorter) 153 which is operable to sort or classify cells contained within vessel 150 into different groups. For example, the cytometer 153 may be configured to classify cells from vessel 150 as one or more of the following: healthy cells, cancer cells, cancel stem cells. In an embodiment, the cytometer 153 is operable to differentiate between either healthy cells and cancerous cells, or cancerous cells and cancer stem cells. Additionally, the vessel 150 and/or cytometer 153 are coupled to the controller 114 such that the controller 114 is used to control the operation of the system (e.g. electrosurgical instrument and electrosurgical generator) based on the classifications or sorting performed by the cytometer 153. Further details of the cytometer 153 are provided below with reference to Fig. 2(b).

A user can interact with the controller 114 via user interface 152, which may be a touch screen display, a membrane keypad and a LCD/LED display, or the like.

The heterodyne detector 126 comprises a mixer 154 arranged to receive a reference signal from a fixed frequency source 156 and a measurement signal from the detector 120 or the detector on the measurement channel (discussed below) via switch 158. After mixing, the output signals are passed through a filter 160 to allow only the lower frequency difference signal to be available for measurement of magnitude and optionally phase using a digital signal processor 162 in a conventional manner. A hardware solution may also be used to enable the magnitude and phase information to be extracted, i.e. a quadrature I-Q mixer may be used. The measurement result is sent to the controller 114, where it is used in subsequent operations associated with the control of the device.

In use, the measurements obtained from the signals produced by detector 120 provide an indication of the amount of power being delivered to the biological tissue at a distal end of the electrosurgical instrument 104. Changes in the delivered power may be indicative of changes in the type of tissue at the distal end of the electrosurgical instrument 104. The controller 114 may select an energy delivery profile based on the measurements. Fundamentally, it is the combination of the microwave frequency and output power level that determines the volume and amount of heating that occurs in the treatment region.

The amount of energy that is reflected by healthy cells may be different to the amount of energy reflected by cancerous cells. The detector may detect this change and the controller may be arranged to recognise that a given change corresponds to the appearance of cancerous cells. The change in the amount of reflected energy may affect the amount of energy transferred into different cell types. The apparatus may be adjustable to account for this. For example, the controller 114 may monitor the amount of delivered energy using the signals from the detector and adjust the output power level if necessary. Dynamic impedance matching may also be implemented to ensure that the reflection coefficient remains as close as possible to zero during the procedure, regardless of any changes in reflection coefficient due to impedance mismatch between the end of the electrosurgical instrument and the contact tissue.

The frequency of the oscillator 108 may be adjustable, e.g. depending on the size of the treatment region. At higher frequencies the depth of penetration is smaller.

The apparatus may be used to assist in a tunnelling process, i.e. the process of inserting the electrosurgical instrument to the treatment region. The electrosurgical instrument may be arranged to radiate microwave energy as the electrosurgical instrument is inserted in order to form a channel for the antenna to be inserted without causing pain, preventing blood loss and reducing the level of discomfort experienced by the patient. In the tunnelling process, it is desirable for the electrosurgical instrument to produce focussed heat with a limited depth of penetration to heat the tissue structures in such a manner that a uniform channel is produced. Since there may be many different tissue structures on the path to the treatment region, sensitivity of the apparatus and dynamic adjustment of the power level may be required. To facilitate this, a measurement channel may be provided between the oscillator 108 and electrosurgical instrument 104. The purpose of the measurement channel is to output low power signals at the electrosurgical instrument which enable properties of any tissue present there to be measured. A power level for a signal through the treatment channel may be selected based on the measurements made using the measurement channel. This arrangement permits a uniform channel to be generated in the tissue.

The output from the splitter 110 on the measurement channel is received by a forward directional coupler 164 connected to couple power from measurement channel. The coupled port of the coupler 164 is connected to a switch 166, whose function is to select either the forward coupled or reflected coupled power under the control of the controller 114 to be conveyed for measurement by the heterodyne detector 126. The output of the forward directional coupler 164 on the measurement channel is received by the first port of a circulator 168, whose function is to isolate the reflected signals travelling back from the electrosurgical instrument 104 from the source 102. Forward signals on the measurement channel travel from the first port of the circulator 168 to its second port, where they are output. Any reflected signals received at the second port of the circulator 168 travel to the third port and are output into a power dump load 170. The output from the second port of the circulator 168 is received by a directional coupler 172, which is configured as a forward power directional coupler and forms a part of a carrier cancellation circuit. The output from directional coupler 172 is fed into the first port of circulator 174. The second port of circulator 174 is connected to the electrosurgical instrument 104 via switch 140. The third port of circulator 174 is connected to the input of a directional coupler 176, which is configured as a forward power directional coupler and forms a part of the carrier cancellation circuit. The function of the circulator 174 is to convey the reflected signal towards the heterodyne detector 126 whilst isolating the reflected signal from the forward signal. Forward signals on the measurement channel travel from the first port of the second circulator 174 to its second port, where they are output. Reflected signals from the electrosurgical instrument 104 are received at the second port of the circulator 174, from where they travel to the third port and are output. The output of the third port of the circulator 174 is received by the directional coupler 176, which is part of the carrier cancellation circuit. After passing through the coupler 176, the reflected signal connected to the switch 166 is conveyed to the heterodyne detector 126 when selected.

The carrier cancellation circuit provides isolation in addition to that provided by the circulators 168, 174. The carrier cancellation circuit comprises the forward directional coupler 172, a phase adjuster 178, an adjustable attenuator 180, and a second forward directional coupler 176. The carrier cancellation circuit works by taking a portion of the forward signal from the coupled port of coupler 172 and adjusting the phase and power level such that it is 180° out of phase out of phase and of the same amplitude as any unwanted signal that gets through to the third port of circulator 174 to enable the unwanted signal component to be cancelled out. The carrier cancellation signal is injected into the output of the third port of circulator 174 using second forward coupler 176.

Since the measurement channel provides reflected signals directly (i.e. not via a coupler) to the heterodyne detector 126 the power delivered on the measurement channel can be much less than that on the treatment channel.

Switches 140, 158 are arranged to switch together to select either the treatment or the measurement channel. The apparatus may periodically switch to the measurement channel during tunnelling to monitor the tissue at the distal end of the electrosurgical instrument. This measurement information may be used to enable appropriate adjustment of the energy profile (power level over specified durations of time) delivered into the biological tissue of interest. It may also be used as the basis for adjustment of the power matching network used to match the impedance of the end of the electrosurgical instrument with the contact tissue, i.e. to ensure that the reflection coefficient is as close as possible to zero.

The arrangements of the directional couplers 122, 134 on the treatment channel provides a further advantage of this embodiment. Conventionally, forward and reverse couplers are inserted in the same path, e.g. between the output of the amplifier and the input to the electrosurgical instrument. This can limit sensitivity of the measurement signals (or the dynamic range of the system) because it is possible for the unwanted signal to be of similar magnitude to the wanted (measurement) signal. This is particularly relevant when the reflected signal is small due to a small mismatch between the antenna and the load impedance. In this invention it may be important to make a measurement in this situation, e.g. where the system impedance is 50Ω and load impedance is 46Ω (i.e. in which 4.17% of the incident power is reflected back). The problem in this case is that an unwanted signal from a decoupled port that travels in the opposite direction from the wanted measurement signal can be of similar magnitude to the wanted signal, thus the measurement signal cannot be discerned from the noise signal. In conventional systems, the isolation between the forward and reverse signals is dependent only upon the coupling factor of the directional coupler (the sampled incident power) and the directivity (how well the coupler distinguishes between the forward and reverse travelling waves) and the total isolation (dB) between the forward and reverse signals equals the sum of the coupling factor (dB) and the directivity (dB).

This problem may be exacerbated if the reflected signal is used to automatically control the energy delivery profile (e.g. via controller 114), because the reflected signal will be corrupted due to the fact that there will always be more forward signal than reflected signal due to path losses between the measurement coupler and the load, i.e. insertion loss of the cable and the antenna/electrosurgical instrument shaft, etc.

The invention may overcome these problems in arrangements where there is no dynamic impedance matching or tuning by relocating the forward and reverse directional couplers to between the output of the power amplifier (or oscillator in the measurement channel) and the input to the first port of the circulator and between the third port of the circulator and the power dump load respectively.

Further increased isolation or enhanced measurement sensitivity between the forward and reverse signals may be achieved by inserting one or more additional circulators (with 50 dump loads connected between the third port and ground) between the forward signal coupler and the first port of the first circulator, with the final circulator being used to measure the reflected signal. Each additional circulator will increase the isolation in terms of the reverse power signal corrupting the forward power signal by the circulator unwanted power flow isolation, i.e. three additional circulators with isolation in unwanted path of 20 dB will increase the overall isolation by 60 dB.

In the treatment mode, the user interface 152 may indicate the energy dosage delivered into the tissue, the treatment time, and any other useful and/or relevant information. It is noted that in treatment mode, the user interface 152 may provide both information on microwave treatment and on electroporation treatment (e,g, pulse width, duty cycle, amplitude, etc.) In biopsy mode, it may be desirable for user interface 152 to show a type of cell detected by the cytometer 153, a level of tissue contained in vessel 150, and when pump 106 has been activated. In measurement mode, it may be desirable for user interface 152 to show or display tissue type and/or tissue state. Also, in biopsy mode and/or measurement mode, may also be desirable to sound an audible alarm or flash the display when cancerous tissue is detected.

As mentioned above, the electrosurgical instrument 104 is also configured to perform electroporation, however, for clarity, the apparatus required to enable the electroporation capabilities is not shown in Fig. 2(a). In an embodiment, the apparatus for performing electroporation is part of the generator as described in WO 2012/076844. This apparatus could be incorporated into the system of Fig. 2(a) in a number of ways. For example, the apparatus could be connected at switch 140, that is, the switch 140 may be modified to have an additional third position for delivering electroporation signals to the electrosurgical instrument 104. Alternatively, an additional switch having two positions could be inserted a distal side of the switch 140, wherein one position connects the flexible transmission cable 142 to switch 140, and the other position connects the flexible transmission cable 142 to the electroporation apparatus. In any case, the system of Fig. 2(a) can be updated to provide an electroporation signal to the electrosurgical instrument 104 in addition to microwave energy. Electroporation signals and microwave energy may be delivered to the electrosurgical instrument 104 separately or simultaneously.

In Fig. 2(a), the cytometer 153 is included as part of the vessel 150. However, in some other embodiments, one or more of the vessel 150, the pump 106, the valve 151, and the cytometer 153 may be combined into the same physical apparatus. For example, the vessel 150 may be a region within the cytometer 153 (e.g. a detection region), and the pump 106 and valve 151 may be parts of the cytometer 153 which function to draw cells from the treatment region into the cytometer 153 for sorting. That is, the vessel 150, the pump 106, the valve 151, and the cytometer 153 may be replaced by a single apparatus which is connected to, and controlled by, the controller 114. This combination (aka cell identification assembly) may have all or some of the capabilities of the vessel 150, the pump 106, the valve 151, and the cytometer 153. Accordingly, the cell identification assembly may be operable to obtain biopsy tissue (e.g. fluid and cells) from a treatment site using the electrosurgical instrument 104. The cell identification assembly may be operable to generate a sample from the biopsy tissue and sort cells of the sample to detect the presence of one or more different cell types, such as, for example, cancer cells, cancer stem cells, healthy cells, non-cancer cells. Further, the cell identification assembly may provide a detection signal to the controller 114 to inform the controller 114 of the presence of particular cell types (e.g. cancerous cells or cancer stem cells). In this way, the controller 114 can perform various operations based on the detection signal, for example, on detecting cancer stem cells, the controller 114 can control the apparatus to perform electroporation using a particular pulse profile for a particular duration, and/or microwave ablation at a particular power for a particular duration. Accordingly, the cell identification assembly provides a mechanism for detecting a particular cell type in biopsy tissue and notifying the controller 114 of the detection, so that the controller 114 can control the system based on the notification.

As mentioned above with reference to Fig. 2(a), some embodiments can include a detector 120 which detects changes in the amount of microwave energy reflected by biological tissue at a treatment site. The controller 114 can then be configured to recognise that a given change corresponds to the appearance of cancerous cells. As such, the detector 120 and controller 114 can perform a first detection stage using microwave energy. Additionally, embodiments include a cell identification assembly which identifies the presence or absence of certain cell types in a biopsy sample using a cytometer 153. As such, the cell identification assembly can perform a second detection stage. For example, the first detection stage can be used to identify cancerous cells from healthy cells, and the second detection stage can be performed on the identified cancerous cells in order to identify cancer stem cells from other cancerous cells. However, in some other embodiments, the second detection stage may be used to confirm the result of the first detection stage. Also, in some other embodiments, the first detection stage is absent and only the second detection stage is present.

In an embodiment, the system of Fig. 2(a) can be additionally configured to deliver fluid (e.g. drugs or chemotherapy) to the treatment region via the conduit in the electrosurgical instrument 102 and the transport pipe 148. In an embodiment, the vessel 150 may contain a compartment (not shown) for storing fluid to be delivered to the treatment region, and one or more valves (not shown) may selectively open and close a first path, from the electrosurgical instrument to the cytometer 153 for tissue extraction, and a second path, from the compartment to the electrosurgical instrument for fluid delivery. The one or more valves may be controllable by the controller 114. The pump 106 may be drivable in a reverse direction in order to inject fluid to the electrosurgical instrument along the second path, or a separate injecting pump may be provided. In another embodiment, the compartment may be separate from the vessel 150 and may join the transport pipe 148 at a switchable junction (not shown). The switchable junction may be controllable by the controller 114 to selectively open and close the first and second paths to enable tissue extraction and fluid delivery. In a further embodiment, a connection between the transport pipe 148 and the vessel 150 or cytometer 153 may be releasable so that the transport pipe 148 can be detached from the vessel 150 or cytometer 153 and then re-attached to the compartment, and vice versa. As such, in an embodiment, the system of Fig. 2(a) has a fluid injection mechanism in fluid communication with the conduit of the electrosurgical instrument 102 such that fluid (e.g. drugs or chemotherapy) can be injected into the treatment region.

### Cytometer instrument

The cytometer (or cell sorter) 153 as described above with reference to Fig. 2(a) will now be described in more detail. In an embodiment, the cytometer 153 may be a flow cytometer which detects and measures physical and chemical characteristics of a population of cells or particles. In another embodiment, the cytometer 153 may be a spectrometer (e.g. a miniature spectrometer), for example, which uses Raman spectroscopy to detect the presence of (or distinguish between) one or more particular cell types.

In an embodiment, the cytometer 153 may include a commercial off-the-shelf cytometer, such as, the DEPArray^{™} System from Menarini-Silicon Biosystems.

In an embodiment, a sample generator may be provided to generate a sample for sorting by the cytometer 153. The sample generator may be part of the vessel 150, the cytometer 153, or may be a separate element connected to both the vessel 150 and cytometer 153. In any case, the sample generator forms a sample for analysis by suspending cells from the vessel 150 in a fluid (e.g. a buffer fluid) and then provides (e.g. injects) the sample to the cytometer 153. The fluid may be provided from a fluid reservoir which may be part of the vessel 150, the cytometer 153, or may be a separate element connected to both the vessel 150 and cytometer 153. In any case, the sample may be focused to ideally flow one cell at a time through a detection region of the cytometer 153 where cell sorting is performed based on the difference of electromagnetic signatures between different cell types (e.g. cancer stem cells vs. other cells). In an embodiment which combines hydro-fluidic and electromagnetic manipulation, cells are dynamically sorted into different physical locations or bins depending on their susceptibility to a specific electromagnetic signal. Specifically, electromagnetic fields in the MHz regime are used to selectively electro-manipulate cells with dielectrophoresis (DEP) forces, as illustrated in Fig. 2(b). Fig. 2(b) shows a sample of cells entering an input region 182 (e.g. a microfluidic channel) of the cytometer 153. The sample includes a suspension containing cancer stem cells and one or more other types of cells. The input region 182 may be configured (e.g. dimensioned) so that cells of the sample flow in substantially single file. The input region 182 is in fluid communication with a detection region 184 such that the sample of cells flow into the detection region 184. The detection region 184 includes a microfluidic channel in-between a first array of electrodes 186 and a second array of electrodes 188. The first array of electrodes 186 is connected to a first drive circuit 187 made up of electronic components (e.g. including an AC source), whereas the second array of electrodes 188 is connected to a second drive circuit 189 made up of electronic components (e.g. including an AC source). The first and second drive circuits may be part of the same electronic circuit. In any case, the first and second drive circuits apply an electromagnetic signal to the first and second arrays to sort the sample of cells into particular locations within a sorting region 190. Specifically, the sorting region 190 includes a first bin 192 and a second bin 194. A cell entering the detection region 184 will be deviated (e.g. moved) by an electromagnetic field generated by the first and second arrays due to the electromagnetic signal applied thereto. The trajectory of a given cell will depend on characteristics of the cell (e.g. whether the cell is a cancer stem cell or not) and the electromagnetic signal. Accordingly, the electromagnetic signal can be selected such that cancer stem cells (e.g. a first predetermined cell type) follow a first trajectory into the first bin 192, whereas non-cancer stem cells (i.e. not the first predetermined cell type) follow a second trajectory into the second bin 194. In this way, the sample of cells entering the cytometer 153 are sorted into different bins. The cytometer 153 can then be used to detect the presence or absence of cancer stem cells in a given sample by determining the presence or absence of cells in the first bin 192. This determination can be performed by the cytometer 153 itself, or by a separate detection apparatus (e.g. which is part of the vessel 150). In any case, a detection signal can be generated to indicate the presence and/or absence of cancer stem cells. This detection signal can be sent to the controller 114 so that operation of the system can be based on the indication.

It is to be understood that in some embodiments only a single bin may be provided, for example, bin 192. In this way, only the cells of a type of interest (e.g. cancel stem cells) can be collected, whereas all other cell types can be discarded.

In an example, during a tunnelling procedure, the electrosurgical instrument 104 may be used (e.g. by a surgeon operating on a patient) with the pump 106 to obtain a first set of biopsy cells in the vessel 150, and the cytometer 153 may be used to identify that the first set of biopsy cells does not include cancer stem cells. In this case, the controller 114 may simply notify the user (e.g. via the user interface 152) that no cancer cells have been detected. Accordingly, the user may have confidence to tunnel the electrosurgical instrument 104 further into the patient. This sequence of operations may be repeated one or more times. At some point, the cytometer 153 may detect the presence of cancer stem cells and transmit a detection signal to the controller 114 to inform the controller 114 of the presence of the cancer stem cells. On receipt of the detection signal, the controller 114 may perform a number of different operations. For example, the controller 114 may notify the user (e.g. via the user interface 152) that cancer stem cells have been detected. Additionally or alternatively, the controller 114 may initiate some form of treatment operation using the electrosurgical instrument 104 and other parts of the system. The treatment operation may include one or more of the following: performing temporary electroporation for a first time period to open the pores of the cancer stem cells (i.e. to sensitise the cancer stem cells); inject drugs (e.g. local chemotherapy) into sensitised cancer stem cells; activate injected drugs using microwave energy; ablate the cancer stem cells using microwave energy for a second time period; and perform irreversible electroporation on the cancer stem cells for a third time period (i.e. to ablate the cells). Of course, whilst the system could be configured to perform such operations automatically (e.g. via the controller 114) on detection of cancer stem cells, it is also possible for the system to only notify the user of the presence of the cancer stem cells (e.g. via the user interface 152) so that the user can perform such operations manually.

The above example concentrates on the cytometer 153 distinguishing between cancer stem cells and other cell types. However, it is to be understood that the cytometer 153 can be configured to distinguish between other cell types or categories. For example, the cytometer 153 can be configured to distinguish between healthy cells and cancerous cells, or between cancerous cells and cancer stem cells, or between blood cells and fat cells. It is noted that, in this context, the expression `configured to' includes selecting a particular electromagnetic signal for applying to the arrays of the detection region 184 which diverts one cell type or category differently to one or more other cell types or categories.

Also, as discussed above, in some other embodiments, one or more of the vessel 150, the pump 106, the valve 151, and the cytometer 153 may be combined into the same physical apparatus. For instance, the vessel 150, the pump 106, the valve 151, and the cytometer 153 may be replaced by a single apparatus (aka cell identification assembly) which is connected to, and controlled by, the controller 114.

### Electrosurgical Instrument

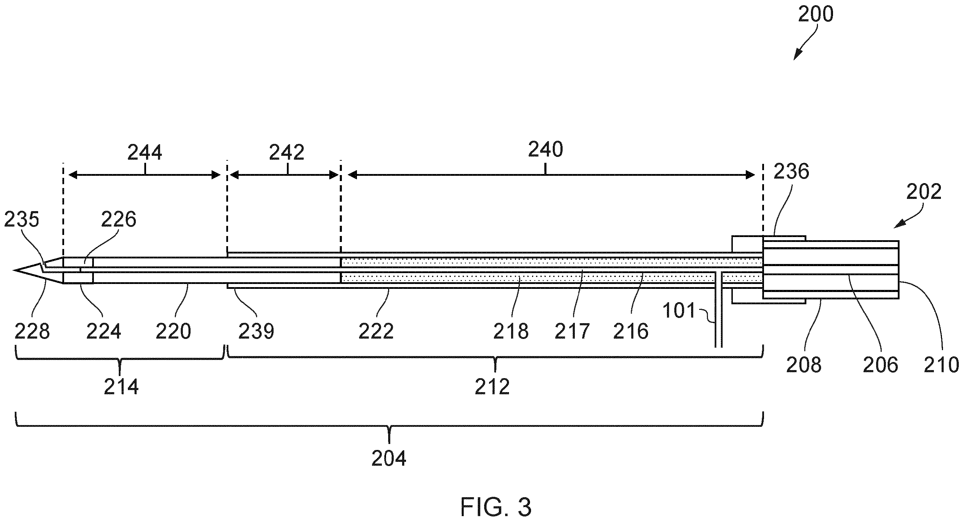

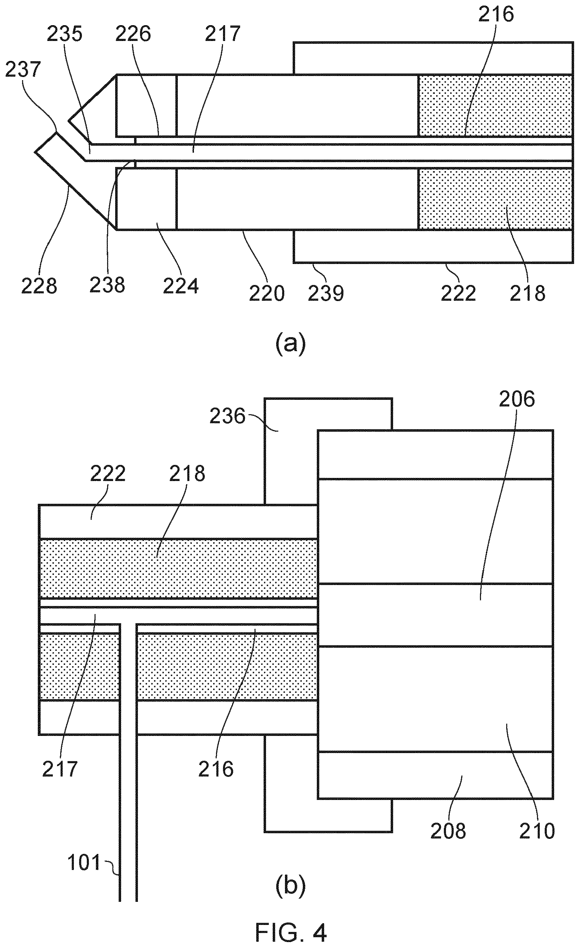

An electrosurgical instrument 200 according to an embodiment of the invention is illustrated in Figs. 3 and 4. Fig. 3 shows a schematic cross-sectional side view of a distal end of electrosurgical instrument 200. Fig. 4a shows an expanded cross-sectional side view of a distal portion of electrosurgical instrument 200, and Fig. 4b shows an expanded cross-sectional side view of a proximal portion of the electrosurgical instrument 200. The electrosurgical instrument 200 may provide the distal assembly 118 of Fig. 1, or the electrosurgical instrument 104 of Fig. 2(a).

Electrosurgical instrument 200 includes a flexible coaxial cable 202 and a radiating tip portion 204 mounted at a distal end of the coaxial cable 202. The coaxial cable 202 may be a conventional flexible 50 Ω coaxial cable suitable for travelling through the instrument channel of a surgical scoping device. The coaxial cable includes a centre conductor 206 and an outer conductor 208 that are separated by a dielectric material 210. The coaxial cable 202 is connectable at a proximal end to an electrosurgical generator to receive microwave energy and an electroporation signal.

The radiating tip portion 204 includes a proximal coaxial transmission line 212 and a distal needle tip 214 mounted at a distal end of the proximal coaxial transmission line 212. The proximal coaxial transmission line 212 comprises an inner conductor 216 that is electrically connected to the centre conductor 206 of the coaxial cable 202 at the distal end of the coaxial cable 202. The inner conductor 216 has a smaller outer diameter than the centre conductor 206, and is made of a material having a high conductivity, e.g. silver.

The inner conductor 216 is surrounded along a proximal portion thereof by a proximal dielectric sleeve 218. The proximal dielectric sleeve may be made of a flexible insulating material, e.g. PTFE or the like. A distal dielectric sleeve 220 is mounted over a distal portion of the inner conductor 216 to form the radiating tip portion 214. The distal dielectric sleeve 220 is formed of a hard insulating material having a higher rigidity than the proximal dielectric sleeve 218. For example, the distal dielectric sleeve 220 may be made of Zirconia.

The proximal coaxial transmission line 212 is completed by an outer conductor 222 mounted around the proximal dielectric sleeve 218. The outer conductor 222 is formed by a flexible tube of conductive material. The tube is configured to have longitudinal rigidity sufficient to transmit a force capable of penetrating biological tissue (e.g. the duodenum wall) whilst also exhibiting suitable lateral flex to enable the instrument to travel through the instrument channel of a surgical scoping device. The inventors have found that nitinol is a particularly suitable material for the outer conductor 222. The nitinol tube may include a conductive coating, e.g. on its inner surface, in order to reduce transmission losses along the proximal coaxial transmission line 212. This coating may be formed by a material having a higher conductivity that the nitinol, e.g. silver or the like.

Inner conductor 216 includes a hollow section defining a bore (or channel or conduit) 217 from the interface between a tissue connection pipe 101 to the distal tip of the radiating tip portion 214, where biological tissue is sucked into inner conductor 216. The inner conductor 216 may also have a solid (i.e. not hollow) section between its connection with centre conductor 206 and its connection with tissue connection pipe 101. The bore 217 of inner conductor 216 has a diameter such that the wall thickness between the solid section and the distal tip of inner conductor 216 is such that the transport of microwave energy is unaffected by the removal of the centre section of the inner conductor 216, and the wall of the hollow section has enough strength to support itself and to allow for the electrosurgical instrument to be assembled with ease when the instrument is manufactured. It is preferable for the thickness of the wall of the hollow section of inner conductor 216 to be at least five or six skin depths in thickness in order to ensure that most of the microwave energy is transferred. The skin depth is determined by the properties of the material and the frequency of operation. For example, the thickness of the wall of the hollow section of inner conductor 216 may be about five microns. The connection pipe 101 connects the bore 217 of inner conductor 216 to the transport pipe 148 of Fig. 2(a), which is attached to collection vessel 150 (or cell identification assembly). The pipe 101 may be made from a dielectric material or a conductor. It is preferable for pipe 101 to be made from a similar material to that of the dielectric sleeve 218 in order to preserve the characteristic impedance of the co-axial structure and to minimise discontinuities within the structure. The location, size and the material used for pipe 101 may affect the transverse electromagnetic (TEM) fields set up in the co-axial structure, but any changes to the field distribution may be compensated for by including a matching transformer inside the structure near pipe 101; the matching transformer may be a tuning stub, which may be a conductive pin or a dielectric post. If a means of matching out the effect of the connection pipe 101 is required, then the matching structure may simply be a change in relative permittivity of dielectric sleeve 218 or an additional pin inserted through the wall of the outer conductor 222 in the region of connection pipe 101. The specific embodiment of the matching structure will be dependent upon the specific geometry of the electrosurgical instrument 200 and it may be necessary to perform an electromagnetic field simulation of the complete electrosurgical instrument to determine the best matching structure to use. It should be noted that for small feed channels 217 and small connection pipes 101, the field discontinuity produced by including the connection pipe 101 into the structure will be negligible and, therefore, it may be ignored. This invention is not limited to the use of a single feed pipe 101. It may be preferable to use a plurality of feed pipes in order to minimise the constriction of flow inside biopsy (or material) channel 217. For example, four feed pipes may be used rather than the single feed pipe 101 shown in Fig. 3. It may be preferable to arrange the four feed pipes such that the total cross-section of the pipes equals the cross-section of the biopsy channel 217 in order to minimise a possible constriction that may occur. In this instance, the biopsy sample (or other material) would be gathered from four outlets (or inlets if material is to be delivered into the body) in the wall of outer conductor 222. The spacing between the feed pipes may be adjusted to minimise the mismatch caused by the introduction of the single feed pipe 101 into the system, i.e. this may remove the need for a separate impedance transformer (or matching stub) to be introduced.

The outer conductor 222 overlays a proximal portion of the distal dielectric sleeve 220, to form a distal portion of the proximal coaxial transmission line 212. The region of overlap may be considered as an intermediate coaxial transmission line. As the distal dielectric sleeve 220 has a higher dielectric constant than the proximal dielectric sleeve 218, the region of overlap between the outer conductor 222 and the distal dielectric sleeve 220 enables a physical length of the radiating tip portion 212 to be reduced whilst maintaining a desired electrical length. The length of the overlap between the outer conductor 222 and the distal dielectric sleeve 220 and the dielectric materials of the distal and proximal dielectric sleeves may be selected to obtain a desired electrical length of the radiating tip portion 212.

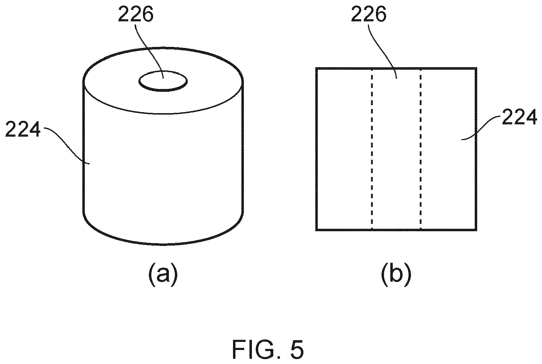

The distal needle tip 214 includes an active electrode 224 mounted at a distal end of the inner conductor 216. The active electrode is a cylindrical piece of conductive material (e.g. brass) having a central channel 226 extending therethrough. The active electrode is illustrated in more detail in Fig. 5, which shows a perspective view of the electrode (a) and a cross-sectional side view of the electrode (b). The distal end of the inner conductor 216 protrudes inside the channel 226, where it is electrically connected to the active electrode 224 (e.g. via a soldered or welded connection, or with a conductive adhesive). An outer diameter of the active electrode substantially matches an outer diameter of the distal dielectric sleeve 220, so that the distal needle tip 214 has a smooth outer surface.

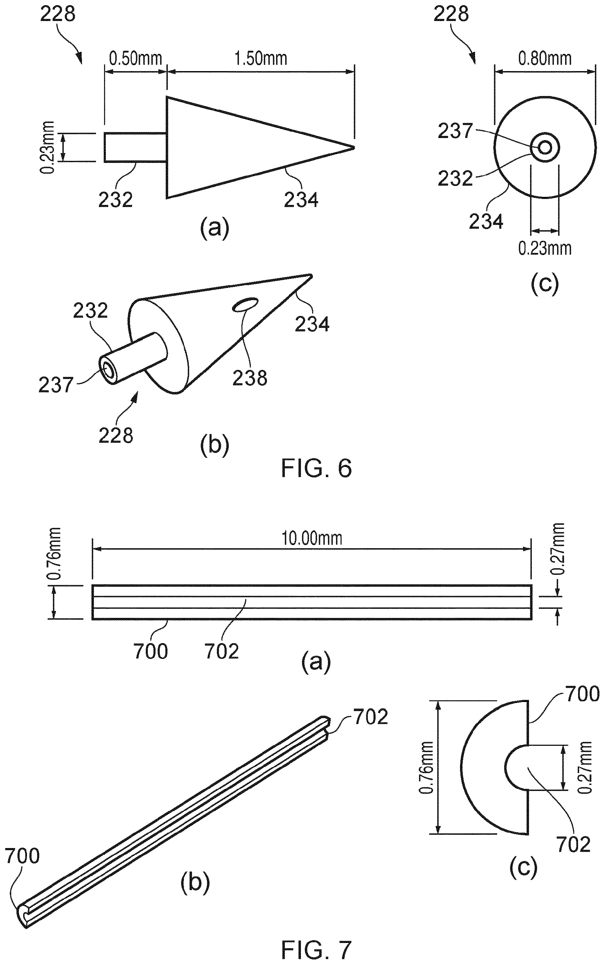

A pointed tip element 228 is mounted on a distal face of the active electrode 224, to facilitate insertion of the instrument into target tissue. The tip element 228 is preferably made of the same material as the distal dielectric sleeve 220 (e.g. Zirconia). The tip element 228 is shown in more detail in Fig. 6, which shows a side view of the tip element (a), a perspective view of the tip element (b), and a rear view of the tip element (c). Example dimensions of the tip element 228 are shown in Figs. 6(a) and 6(c). The tip element 228 has a conical body 230 having a protrusion 232 extending from a proximal side thereof. The protrusion 232 is shaped to fit inside the channel 226 in the active electrode 224, to hold the tip element 228 in place. The tip element 228 may be secured to the active electrode 224, e.g. using an adhesive. The tip element 228 also has a channel 235 extending therethrough. As seen more particularly on Fig. 6b, the channel 235 has an inlet 237 and an outlet 238. The inlet 237 is arranged (e.g. sized and positioned) to align with the distal end of bore 217 of inner conductor 216 such that the channel 235 provides an extension to the bore 217. The outlet 238 is shown on a side portion of tip element 224, however, it is to be understood that in some other embodiments, the outlet 238 may be located at the apex of tip element 228. As such, the channel 235 may or may not have the bend shown in Figs. 3 and 4a.

The proximal dielectric sleeve 218 and the distal dielectric sleeve 220 may be formed as tubes that slide over the inner conductor 216. In one embodiment, the distal dielectric sleeve 220 may be composed of a pair of cooperating parts which are mounted around the inner conductor 216. Fig. 7 shows an example of a part 700 that may be used to form the distal dielectric sleeve 220. Fig. 7 shows a side view of the part (a), a perspective view of the part (b) and a front view of the part (c). Example dimensions of the part 700 are shown in Figs. 7(a) and 7(c). The part 700 is a semicylindrical piece of rigid dielectric material (e.g. Zirconia) having a longitudinal groove 702 extending along its length. A pair of parts 700 may be assembled together to form the distal dielectric sleeve 220, so that the grooves 702 in each part 700 together form a channel in which the inner conductor 216 is received. The two parts 700 may be secured together, e.g. using an adhesive. Such a structure of the distal dielectric sleeve 220 may facilitate assembly of the radiating tip portion 212. A similar structure comprising a pair of cooperating parts may also be used for the proximal dielectric sleeve 218.

The proximal coaxial transmission line 212 is secured to the distal end of the coaxial cable 202 by a collar (or connector) 236. The collar 236 may act as a radial crimp to secure the proximal coaxial transmission line 212 in place. The collar 236 is also arranged to electrically connect the outer conductor 208 of the coaxial cable 202 to the outer conductor 218 of the proximal coaxial transmission line 212. The collar 236 is thus formed from a conductive material, e.g. brass or the like. The collar 236 may provide at least part of the connector 144 of Fig. 2(a).

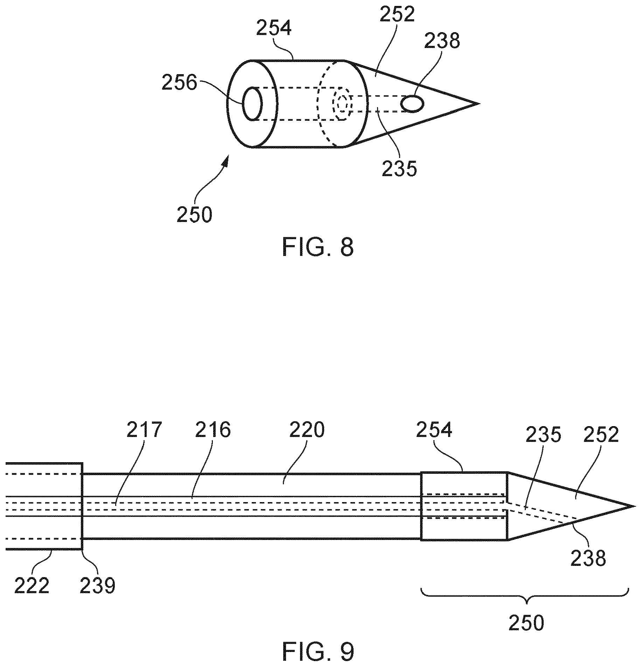

Figs. 8 and 9 show an alternative arrangement for the distal tip. In this arrangement the pointed tip element and active electrode are combined in a single tip element 250. The tip element 250 comprises a distal pointed tip 252, e.g. having a conical shape, formed integrally with a proximal cylindrical portion 254 that has a bore 256 therein for receiving a distal portion of the inner conductor 216. As before, the tip element 250 has internal channel 235 which connects the hollow inside of inner conductor 216 (i.e. the bore 217) with the outlet 238. The tip element 250 may be fabricated from a single piece of conductive material, such as silver. In use, microwave energy and energy having an electroporation waveform may be conveyed from the coaxial cable 202 to the radiating tip portion. Energy received from the coaxial cable 202 may be transmitted along the proximal coaxial transmission line 212 to the distal needle tip 214, where it may be delivered to target tissue.

At microwave energies, the distal needle tip 214 is arranged to perform as a half wavelength transformer for delivery of the microwave energy into target tissue. In other words, an electrical length of the distal needle tip 214 may correspond to half a wavelength of the microwave energy. In this manner, microwave energy may be efficiently delivered to target tissue, in order to ablate the target tissue.

The microwave energy may be delivered in pulses in order to minimise heating in the radiating tip portion 212 during microwave ablation. The inventors have found that the energy delivery cycles or profiles listed below may enable efficient delivery of microwave energy whilst minimising heating in the radiating tip portion 212, however other energy delivery cycles are also possible:
- 10 ms microwave energy delivery followed by 90 ms off (i.e. with no microwave energy delivery);
- 10 ms microwave energy delivery followed by 50 ms off;
- 10 ms microwave energy delivery followed by 30 ms off;
- 100 ms microwave energy delivery followed by 900 ms off;
- 100 ms microwave energy delivery followed by 500 ms off;
- 100 ms microwave energy delivery followed by 300 ms off;

When electroporation energy is conveyed to the radiating tip portion, an electric field may be set up between the active electrode 224 and a distal portion 239 (distal end) of the outer conductor 222. In this manner, a distalmost edge or end termination of the outer conductor 222 (which may be exposed) may behave as a return electrode for the electroporation energy. The electric field may cause electroporation (e.g. irreversible electroporation) of tissue located around the distal needle tip 214. As the active electrode 224 is disposed substantially symmetrically about a longitudinal axis of the instrument, the electric field caused by the electroporation waveform may be axially symmetrical. In other examples, the treatment region may be non-symmetrical, e.g. through suitable configuration of the active electrode.

The electrosurgical instrument 200 is configured for use as an ablation device to deliver microwave and electroporation energy conveyed along the coaxial cable into biological tissue. The electrosurgical instrument 200 is designed in particular to be suitable for insertion through an instrument channel of a surgical scoping device (e.g. an endoscopic ultrasound (EUS) apparatus) to a treatment site. The treatment site may be the pancreas, whereby an instrument cord of the surgical scoping device is inserted into the duodenum, whereupon the electrosurgical instrument 200 is extended to penetrate through the wall of the duodenum into the pancreas to treatment.

The electrosurgical instrument may have several features that render it suitable for use in this context. The radiating tip portion 212 of the instrument desirably has a length equal to or greater than 40 mm with a maximum outer diameter of about 19G (1.067mm) or 22G (0.7176mm). This can ensure the needle is long enough to reach tumours, for example, located within the pancreas, and can ensure that the penetration hole is not too large, to facilitate healing.

Fig. 3 shows example dimensions of electrosurgical instrument 200. In a first example, the dimension indicated by reference numeral 240, which corresponds to a length of the proximal dielectric sleeve 218, may be 37.0 mm. The dimension indicated by reference numeral 242, which corresponds to a length of the overlap between the outer conductor 222 and the distal dielectric sleeve 220, may be 4.70 mm. The dimension indicated by reference numeral 244, which corresponds to a distance from the distal end of the outer conductor 222 to the distal end of the active electrode 224, may be 3.00 mm. In a second example, which uses the tip element shown in Fig. 9, the dimension 240 is 37.0 mm, the dimension 242 is 8.30 mm, and the dimension 244 is 5.00 mm.

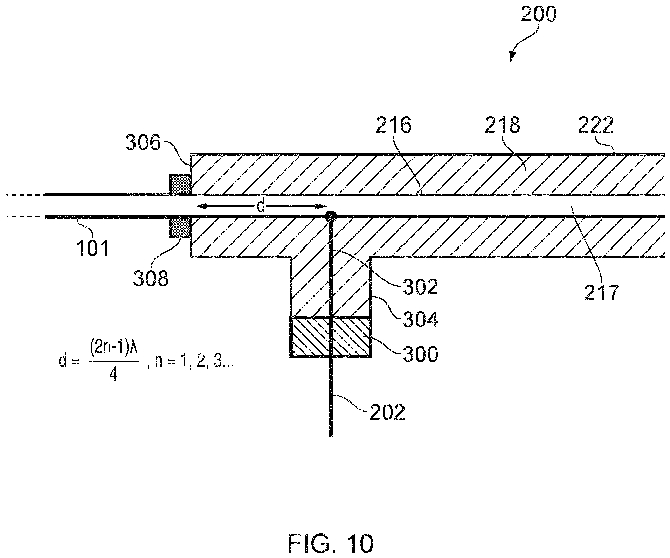

Fig. 10 shows an alternative arrangement for the proximal end of the electrosurgical instrument 200. In this embodiment, the distal end of the electrosurgical instrument 200 is as presented in above-described Fig. 4(a). It is noted that the distal end is located to the right of Fig. 10 (i.e. opposite to Fig. 4(b)). As stated above, electrosurgical instrument 200 has a coaxial feed structure that comprises the outer conductor 222 separated from the inner conductor 216 by the dielectric material 218. The inner conductor 216 is hollow to define the channel 217 for removing biopsy tissue (e.g. cells or fluid) from a treatment site at the distal end of the instrument. However, in this alternative arrangement, the feed structure is side-fed, i.e. the microwave energy is delivered into the instrument 200 from a direction that is angled with respect to the axis of the feed structure, i.e. 90° to the axis. As before, the microwave energy is delivered from a cable assembly 202; however, this time the cable 202 is connected to the instrument 200 via a connector 300. The connector 300 may form part of the connector 144 of Fig. 2(a). The connector 300 may be conventional, e.g. N-type, SMA-type or and MCX. The connector 300 has a centre pin 302 that extends from the connector 300 through the dielectric material 218 to contact the inner conductor 216. The connector 300 also has a conducting outer sleeve 304 in electrical contact with the outer conductor 222. To ensure the energy feed is efficient, the inner conductor 216 (302) and outer conductor 222 (304) are brought into electrical contact with each other at a proximal end 306 of the instrument 200 to create a short circuit condition, and the centre pin 302 contacts the inner conductor 216 at a distance that is an odd multiple of a quarter wavelength from the short circuit location to produce an E-field maximum at this point. This is shows as reference sign `d' in Fig. 10.

An advantage of the side-fed arrangement is that the biopsy tissue (e.g. fluid or cells) can be extracted along the axis of the coaxial structure, e.g. through the flexible extraction tube 101 attached at the proximal end 306 of the instrument 200. The extraction path may thus be free from sharp corners, which may facilitate smooth flow. A plug 308 may be attached to seal around the interface between the instrument 200 and extraction tube 101 to prevent leakage.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An electrosurgical system, comprising:
an electrosurgical generator (102) arranged to supply microwave energy and an electroporation signal;
an electrosurgical instrument (104) for inserting to a treatment region in biological tissue, the electrosurgical instrument comprising:
a coaxial cable connected to the electrosurgical generator to receive the microwave energy and the electroporation signal,
a rod-shaped radiating tip portion coupled to a distal end of the coaxial cable to receive the microwave energy and the electroporation signal, the radiating tip portion being configured to radiate the microwave energy from its distal end into the treatment region and to establish an electric field at its distal end using the electroporation signal to electroporate biological tissue in the treatment region, and
a conduit (148) configured to convey biological tissue away from the treatment region; and
a cytometer (150) in fluid communication with the conduit to receive biological tissue, the cytometer being configured to detect the presence of a first predetermined cell type in the received biological tissue.

2. The electrosurgical system of claim 1, wherein the radiating tip portion comprises:
a proximal coaxial transmission line configured to receive and convey the microwave energy, the proximal coaxial transmission line including an inner conductor, an outer conductor and a dielectric material separating the inner conductor from the outer conductor; and
a distal needle tip mounted at a distal end of the proximal coaxial transmission line, the distal needle tip comprising a rigid dielectric sleeve that extends the longitudinal direction from a distal end of the proximal coaxial transmission line,
wherein the rod-shaped radiating tip portion has a diameter less than a diameter of the coaxial cable,
wherein the rigid dielectric sleeve surrounds an elongate conductive element that is electrically connected to the inner conductor of the proximal coaxial transmission line and extends beyond a distal end of the outer conductor of the proximal coaxial transmission line, wherein the elongate conductive element is configured to operate as a half wavelength transformer for the microwave energy to thereby radiate the microwave energy from the distal needle tip into biological tissue,
wherein the elongate conductive element terminates at an active electrode exposed on a distal end of distal needle tip, and
wherein the active electrode is axially spaced from a return electrode that is electrically connected to the distal end of the outer conductor of the proximal coaxial transmission line, the active electrode and return electrode being configured to establish the electric field for electroporation of biological tissue at the distal needle tip.

3. An electrosurgical system according to claim 2, wherein the dielectric material of the proximal coaxial transmission line is more flexible than the rigid dielectric sleeve.

4. An electrosurgical system according to claim 2 or 3, wherein the active electrode is a conductive ring arranged concentrically with the elongate conductive element.

5. An electrosurgical system according to claim 4, wherein the conductive ring has a channel extending longitudinally therethrough, and wherein a portion of the elongate conductive element is contained within the channel.

6. An electrosurgical system according to claim 5, wherein the distal needle tip comprises a tip element mounted at a distal end of the conductive ring, and wherein a distal end of the tip element is pointed.

7. An electrosurgical system according to any of claims 2 to 6, wherein a distal portion of the outer conductor overlays a proximal portion of the rigid dielectric sleeve.

8. An electrosurgical system according to any of claims 2 to 7, wherein the conduit includes a bore in the inner conductor and the elongate conductive element, and wherein the conduit includes at least one pipe connected to the inner conductor for conveying the biological tissue away from the bore.

9. An electrosurgical system of claim 8, wherein the conduit includes an outlet on the axis of the proximal coaxial transmission line and the microwave energy and electroporation signal are connected to the radiating tip portion at an angle to the axis of the proximal coaxial transmission line.

10. An electrosurgical system of claim 8, wherein the microwave energy and the electroporation signal are fed into the radiating tip portion along the axis of the proximal coaxial transmission line and the biological tissue is extracted using the at least one pipe that is angled to the axis of the proximal coaxial transmission line.

11. An electrosurgical system according to any preceding claim, wherein the conduit is integrated with the radiating tip portion.

12. An electrosurgical system according to any preceding claim, further comprising a surgical scoping device having a flexible insertion cord for insertion into a patient's body, wherein the flexible insertion cord has an instrument channel running along its length, and wherein the electrosurgical instrument is dimensioned to fit within the instrument channel.

13. An electrosurgical system according to any preceding claim, further comprising:
a detector configured to detect microwave power reflected back from the treatment region, and
a controller configured to detect a second predetermined cell type in biological tissue in the treatment region based on changes in the detected reflected microwave power.

14. An electrosurgical system according to any preceding claim, further comprising an impedance matching mechanism arranged to match the impedance of the electrosurgical generator with the radiating tip portion.

15. The electrosurgical system of any preceding claim, further comprising a cell identification assembly comprising:
the cytometer,
a suction pump in fluid communication with the conduit to extract biological tissue thereform,
a sample generator for suspending cells of the extracted biological tissue in a fluid to generate a sample,
wherein the cytometer is configured to detect the presence of the first predetermined cell type using the sample.

16. The electrosurgical system of any preceding claim, further comprising a fluid injecting mechanism in fluid communication with the conduit, the fluid injecting mechanism being configured to inject fluid into the treatment region.

## Patentansprüche

1. Elektrochirurgisches System, das Folgendes umfasst:
einen elektrochirurgischen Generator (102), der angeordnet ist, um Mikrowellenenergie und ein Elektroporationssignal zuzuführen;
ein elektrochirurgisches Instrument (104) zum Einbringen in eine Behandlungsregion in biologischem Gewebe, wobei das elektrochirurgische Instrument Folgendes umfasst:
ein Koaxialkabel, das mit dem elektrochirurgischen Generator verbunden ist, um die Mikrowellenenergie und das Elektroporationssignal zu empfangen,
einen stabförmigen Strahlungsspitzenabschnitt, der mit einem distalen Ende des Koaxialkabel gekoppelt ist, um die Mikrowellenenergie und das Elektroporationssignal zu empfangen, wobei der Strahlungsspitzenabschnitt ausgelegt ist, um die Mikrowellenenergie von seinem distalen Ende in die Behandlungsregion abzustrahlen und ein elektrisches Feld an seinem distalen Ende unter Verwendung des Elektroporationssignals zu erzeugen, um biologisches Gewebe in der Behandlungsregion zu elektroporieren, und
eine Leitung (148), die ausgelegt ist, um biologisches Gewebe von der Behandlungsregion wegzuleiten; und
ein Zytometer (150), das in Fluidkommunikation mit der Leitung steht, um biologisches Gewebe zu empfangen, wobei das Zytometer ausgelegt ist, um das Vorliegen eines ersten vorbestimmten Zelltyps in dem empfangenen biologischen Gewebe zu detektieren.

2. Elektrochirurgisches System nach Anspruch 1, wobei der Strahlungsspitzenabschnitt Folgendes umfasst:
eine proximale koaxiale Übertragungsleitung, die ausgelegt ist, um die Mikrowellenenergie zu empfangen und zu übertragen, wobei die proximale koaxiale Übertragungsleitung einen Innenleiter, einen Außenleiter und ein dielektrisches Material, das den Innenleiter vom Außenleiter trennt, umfasst; und
eine distale Nadelspitze, die an einem distalen Ende der proximalen koaxialen Übertragungsleitung montiert ist, wobei die distale Nadelspitze eine starre dielektrische Ummantelung umfasst, die sich von einem distalen Ende der proximalen koaxialen Übertragungsleitung aus in Längsrichtung erstreckt,
wobei der stabförmige Strahlungsspitzenabschnitt einen Durchmesser aufweist, der kleiner als der Durchmesser des Koaxialkabels ist,
wobei die starre dielektrische Ummantelung ein längliches leitfähiges Element umgibt, das elektrisch mit dem Innenleiter der proximalen koaxialen Übertragungsleitung verbunden ist und sich über ein distales Ende des Außenleiters der proximalen koaxialen Übertragungsleitung hinaus erstreckt, wobei das längliche leitfähige Element ausgelegt ist, als Halbwellenlängentransformator für die Mikrowellenenergie zu fungieren, um dadurch die Mikrowellenenergie von der distalen Nadelspitze in biologisches Gewebe abzustrahlen,
wobei das längliche leitfähige Element mit einer aktiven Elektrode endet, die auf dem distalen Ende der distalen Nadelspitze freigelegt ist, und
wobei die aktive Elektrode von einer Rückführungselektrode, die elektrisch mit dem distalen Ende des Außenleiters der proximalen koaxialen Übertragungsleitung verbunden ist, axial beabstandet ist, wobei die aktive Elektrode und die Rückführungselektrode ausgelegt sind, um das elektrische Feld zur Elektroporation von biologischem Gewebe an der distalen Nadelspitze zu erzeugen.

3. Elektrochirurgisches System nach Anspruch 2, wobei das dielektrische Material der proximalen koaxialen Übertragungsleitung biegsamer ist als die starre dielektrische Ummantelung.

4. Elektrochirurgisches System nach Anspruch 2 oder 3, wobei die aktive Elektrode ein leitfähiger Ring ist, der konzentrisch mit dem länglichen leitfähigen Element angeordnet ist.

5. Elektrochirurgisches System nach Anspruch 4, wobei der leitfähige Ring einen Kanal aufweist, der sich der Länge nach durch ihn hindurch erstreckt, und wobei ein Abschnitt des länglichen leitfähigen Elements innerhalb des Kanals enthalten ist.

6. Elektrochirurgisches System nach Anspruch 5, wobei die distale Nadelspitze ein Spitzenelement umfasst, das an einem distalen Ende des leitfähigen Rings montiert ist, und wobei ein distales Ende des Spitzenelements zugespitzt ist.

7. Elektrochirurgisches System nach einem der Ansprüche 2 bis 6, wobei ein distaler Abschnitt des Außenleiters über einem proximalen Abschnitt der starren dielektrischen Ummantelung liegt.

8. Elektrochirurgisches System nach einem der Ansprüche 2 bis 7, wobei die Leitung eine Bohrung im Innenleiter und im länglichen leitfähigen Element umfasst und wobei die Leitung zumindest einen Schlauch umfasst, der mit dem Innenleiter verbunden ist, um das biologische Gewebe von der Bohrung wegzuleiten.

9. Elektrochirurgisches System nach Anspruch 8, wobei die Leitung einen Auslass auf der Achse der proximalen koaxialen Übertragungsleitung umfasst und die Mikrowellenenergie und das Elektroporationssignal mit dem Strahlungsspitzenabschnitt in Bezug auf die Achse der proximalen koaxialen Übertragungsleitung abgewinkelt verbunden sind.

10. Elektrochirurgisches System nach Anspruch 8, wobei die Mikrowellenenergie und das Elektroporationssignal entlang der Achse der proximalen koaxialen Übertragungsleitung in den Strahlungsspitzenabschnitt zugeführt werden und das biologische Gewebe unter Verwendung des zumindest einen Schlauchs, der in Bezug auf die Achse der proximalen koaxialen Übertragungsleitung abgewinkelt ist, extrahiert wird.

11. Elektrochirurgisches System nach einem der vorangegangenen Ansprüche, wobei die Leitung mit dem Strahlungsspitzenabschnitt einstückig verbunden ist.

12. Elektrochirurgisches System nach einem der vorangegangenen Ansprüche, das ferner eine chirurgische Sondierungsvorrichtung mit einem biegsamen Einführungskabel zum Einführen in den Körper eines Patienten umfasst, wobei das biegsame Einführungskabel einen entlang seiner Länge verlaufenden Instrumentenkanal aufweist und wobei das elektrochirurgische Instrument bemessen ist, um in den Instrumentenkanal zu passen.

13. Elektrochirurgisches System nach einem der vorangegangenen Ansprüche, das ferner Folgendes umfasst:
einen Detektor, der ausgelegt ist, um Mikrowellenleistung, die von der Behandlungsregion zurückreflektiert wird, zu detektieren, und
eine Steuerung, die ausgelegt ist, um einen zweiten vorbestimmten Zelltyp in biologischem Gewebe in der Behandlungsregion basierend auf Änderungen in der detektierten reflektierten Mikrowellenleistung zu detektieren.

14. Elektrochirurgisches System nach einem der vorangegangenen Ansprüche, ferner einen Impedanzanpassungsmechanismus umfassend, der angeordnet ist, um die Impedanz des elektrochirurgischen Generators an den Strahlungsspitzenabschnitt anzupassen.

15. Elektrochirurgisches System nach einem der vorangegangenen Ansprüche, ferner eine Zellidentifikationsanordnung umfassend, die Folgendes umfasst:
das Zytometer,
eine Ansaugpumpe, die in Fluidkommunikation mit der Leitung steht, um biologisches Gewebe daraus zu extrahieren,
einen Probengenerator zur Suspension von Zellen des extrahierten biologischen Gewebes in einem Fluid, um eine Probe zu erzeugen,
wobei das Zytometer ausgelegt ist, um die Gegenwart des ersten vorbestimmten Zelltyps unter Verwendung der Probe zu detektieren.

16. Elektrochirurgisches System nach einem der vorangegangenen Ansprüche, ferner einen Fluidinjektionsmechanismus umfassend, der in Fluidkommunikation mit der Leitung steht, wobei der Fluidinjektionsmechanismus ausgelegt ist, um Fluid in die Behandlungsregion zu injizieren.

## Revendications

1. Système électrochirurgical, comprenant :
un générateur électrochirurgical (102) conçu pour fournir de l'énergie micro-onde et un signal d'électroporation ;
un instrument électrochirurgical (104) destiné à être inséré dans une région de traitement dans un tissu biologique, l'instrument électrochirurgical comprenant :
un câble coaxial connecté au générateur électrochirurgical pour recevoir l'énergie micro-onde et le signal d'électroporation,
une partie pointe de rayonnement en forme de tige couplée à une extrémité distale du câble coaxial pour recevoir l'énergie micro-onde et le signal d'électroporation, la partie pointe de rayonnement étant configurée pour rayonner l'énergie micro-onde depuis son extrémité distale dans la région de traitement et pour établir un champ électrique au niveau de son extrémité distale à l'aide du signal d'électroporation pour électroporer un tissu biologique dans la région de traitement, et
un conduit (148) configuré pour transporter un tissu biologique à l'opposé de la région de traitement ; et
un cytomètre (150) en communication fluidique avec le conduit pour recevoir un tissu biologique, le cytomètre étant configuré pour détecter la présence d'un premier type de cellule prédéterminé dans le tissu biologique reçu.

2. Système électrochirurgical selon la revendication 1, dans lequel la partie pointe de rayonnement comprend :
une ligne de transmission coaxiale proximale configurée pour recevoir et transporter l'énergie micro-onde, la ligne de transmission coaxiale proximale incluant un conducteur interne, un conducteur externe et un matériau diélectrique séparant le conducteur interne du conducteur externe ; et
une pointe d'aiguille distale montée au niveau d'une extrémité distale de la ligne de transmission coaxiale proximale, la pointe d'aiguille distale comprenant un manchon diélectrique rigide qui s'étend dans la direction longitudinale depuis une extrémité distale de la ligne de transmission coaxiale proximale,
dans lequel la partie pointe de rayonnement en forme de tige a un diamètre inférieur à un diamètre du câble coaxial,
dans lequel le manchon diélectrique rigide entoure un élément conducteur allongé qui est connecté électriquement au conducteur interne de la ligne de transmission coaxiale proximale et s'étend au-delà d'une extrémité distale du conducteur externe de la ligne de transmission coaxiale proximale, dans lequel l'élément conducteur allongé est configuré pour fonctionner comme un transformateur demi longueur d'onde pour l'énergie micro-onde pour rayonner ainsi l'énergie micro-onde de la pointe d'aiguille distale dans un tissu biologique,
dans lequel l'élément conducteur allongé se termine au niveau d'une électrode active exposée sur une extrémité distale de pointe d'aiguille distale, et
dans lequel l'électrode active est espacée axialement d'une électrode de retour qui est connectée électriquement à l'extrémité distale du conducteur externe de la ligne de transmission coaxiale proximale, l'électrode active et l'électrode de retour étant configurées pour établir le champ électrique pour l'électroporation du tissu biologique au niveau de la pointe d'aiguille distale.

3. Système électrochirurgical selon la revendication 2, dans lequel le matériau diélectrique de la ligne de transmission coaxiale proximale est davantage flexible que le manchon diélectrique rigide.

4. Système électrochirurgical selon la revendication 2 ou 3, dans lequel l'électrode active est un anneau conducteur agencé de manière concentrique avec l'élément conducteur allongé.

5. Système électrochirurgical selon la revendication 4, dans lequel l'anneau conducteur présente un canal s'étendant longitudinalement à travers lui, et dans lequel une partie de l'élément conducteur allongé est contenue à l'intérieur du canal.

6. Système électrochirurgical selon la revendication 5, dans lequel la pointe d'aiguille distale comprend un élément pointe monté au niveau d'une extrémité distale de l'anneau conducteur, et dans lequel une extrémité distale de l'élément pointe est pointue.

7. Système électrochirurgical selon l'une quelconque des revendications 2 à 6, dans lequel une partie distale du conducteur externe se superpose à une partie proximale du manchon diélectrique rigide.

8. Système électrochirurgical selon l'une quelconque des revendications 2 à 7, dans lequel le conduit inclut un alésage dans le conducteur interne et l'élément conducteur allongé, et dans lequel le conduit inclut au moins un tuyau relié au conducteur interne pour transporter le tissu biologique à l'opposé de l'alésage.

9. Système électrochirurgical selon la revendication 8, dans lequel le conduit inclut une sortie sur l'axe de la ligne de transmission coaxiale et l'énergie micro-onde et le signal d'électroporation sont connectés à la partie pointe de rayonnement en formant un angle par rapport à l'axe de la ligne de transmission coaxiale proximale.

10. Système électrochirurgical selon la revendication 8, dans lequel l'énergie micro-onde et le signal d'électroporation sont fournis à la partie pointe de rayonnement le long de l'axe de la ligne de transmission coaxiale proximale et le tissu biologique est extrait à l'aide d'au moins un tuyau qui forme un angle par rapport à la ligne de transmission coaxiale proximale.

11. Système électrochirurgical selon une quelconque revendication précédente, dans lequel le conduit est intégré à la partie pointe de rayonnement.

12. Système électrochirurgical selon une quelconque revendication précédente, comprenant en outre un dispositif d'endoscopie chirurgicale ayant une corde d'insertion flexible destinée à être insérée dans le corps d'un patient, dans lequel la corde d'insertion flexible présente un canal d'instrument parcourant sa longueur, et dans lequel l'instrument électrochirurgical est dimensionné pour s'adapter à l'intérieur du canal d'instrument.

13. Système électrochirurgical selon une quelconque revendication précédente, comprenant en outre :
un détecteur configuré pour détecter une puissance micro-onde renvoyée par la région de traitement, et
une unité de commande configurée pour détecter un second type de cellule prédéterminé dans un tissu biologique dans la région de traitement sur la base de changements de la puissance micro-onde réfléchie détectée.

14. Système électrochirurgical selon une quelconque revendication précédente, comprenant en outre un mécanisme d'adaptation d'impédance conçu pour adapter l'impédance du générateur électrochirugical à la partie pointe de rayonnement.

15. Système électrochirurgical selon une quelconque revendication précédente, comprenant en outre un ensemble d'identification de cellule comprenant :
le cytomètre,
une pompe d'aspiration en communication fluidique avec le conduit pour extraire un tissu biologique de celui-ci,
un générateur d'échantillon pour mettre en suspension des cellules du tissu biologique extrait dans un fluide pour générer un échantillon,
dans lequel le cytomètre est configuré pour détecter la présence du premier type de cellule prédéterminé à l'aide de l'échantillon.

16. Système électrochirurgical selon une quelconque revendication précédente, comprenant en outre un mécanisme d'injection de fluide en communication fluidique avec le conduit, le mécanisme d'injection de fluide étant configuré pour injecter du fluide dans la région de traitement.
